(19) Europäisches Patentamt
European Patent Office
Office européen des brevets

(11) **EP 2 793 692 B1**

(12) **FASCICULE DE BREVET EUROPEEN**

(45) Date de publication et mention de la délivrance du brevet:
**01.11.2023  Bulletin 2023/44**

(21) Numéro de dépôt: **12810290.2**

(22) Date de dépôt: **21.12.2012**

(51) Classification Internationale des Brevets (IPC):
**A61B 5/0215** $^{(2006.01)}$

(52) Classification Coopérative des Brevets (CPC):
**A61B 5/0215;** A61B 2560/0266; A61B 2560/066; A61B 2562/0247; A61B 2562/225

(86) Numéro de dépôt international:
**PCT/EP2012/076582**

(87) Numéro de publication internationale:
**WO 2013/092969 (27.06.2013 Gazette 2013/26)**

(54) **PROCEDE ET DISPOSITIF DE SURVEILLANCE DE LA MESURE DE LA PRESSION ARTERIELLE PAR CATHETERISME ARTERIEL D'UN PATIENT**

VERFAHREN UND VORRICHTUNG ZUR ÜBERWACHUNG EINER BLUTDRUCKMESSUNG DURCH ARTERIELLE KATHETERISIERUNG EINES PATIENTEN

METHOD AND DEVICE FOR MONITORING BLOOD PRESSURE MEASUREMENT BY ARTERIAL CATHETERIZATION OF A PATIENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2011  FR 1162302**

(43) Date de publication de la demande:
**29.10.2014  Bulletin 2014/44**

(73) Titulaires:
• **Université Grenoble Alpes**
  **38400 Saint Martin d'Hères (FR)**
• **Automatisme et Informatique Industrielle**
  **74960 Cran Gevrier (FR)**

(72) Inventeurs:
• **GRIMBERT, Francis**
  **38330 Biviers (FR)**
• **LAVAULT, Yves**
  **74960 Cran Gevrier (FR)**

(74) Mandataire: **Regimbeau**
  **87, rue de Sèze**
  **69451 Lyon Cedex 06 (FR)**

(56) Documents cités:
DE-A1- 3 927 990          US-A1- 2004 024 294
US-A1- 2006 064 021      US-A1- 2010 094 113
US-A1- 2010 241 013      US-A1- 2010 331 708

• **CLAUDE PROMONET ET AL: "Time-dependent Pressure Distortion in a Catheter Transducer System0", ANESTHESIOLOGY, AMERICAN SOCIETY OF ANESTHESIOLOGISTS, PHILADELPHIA, PA, US, vol. 92, no. 1, 1 janvier 2000 (2000-01-01), pages 208-218, XP007920970, ISSN: 0003-3022**

EP 2 793 692 B1

**Description**

**DOMAINE DE L'INVENTION**

La présente invention concerne un procédé et un dispositif de surveillance et, le cas échéant, de correction en continu de la mesure de la pression artérielle par cathétérisme artériel d'un patient.

**ARRIERE PLAN DE L'INVENTION**

Les mesures de pression artérielle par cathétérisme artériel, notamment de l'artère radiale, d'un patient sont une pratique courante en service de réanimation et de soins intensifs, en bloc opératoire de chirurgie lourde et en cardiologie interventionnelle.

En effet, cette technique permet d'une part de surveiller en continu la pression artérielle qui, en raison de la pathologie du patient, est susceptible de varier brutalement et dans des proportions importantes.

Par ailleurs, cette technique est compatible avec des prélèvements réguliers de sang artériel pour l'analyse des gaz du sang et des examens biologiques.

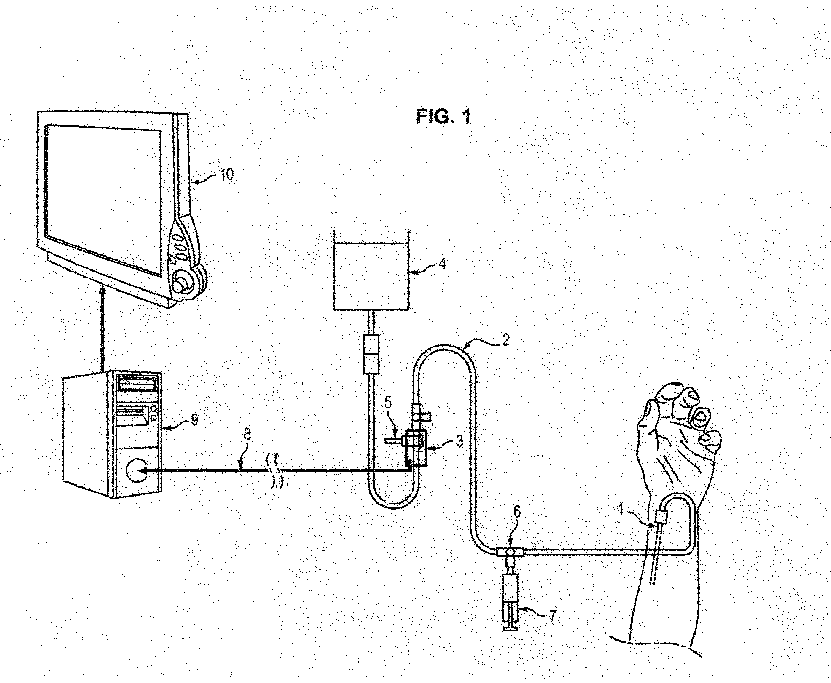

La figure 1 illustre un dispositif actuellement très utilisé pour la mesure de la pression artérielle par cathétérisme de l'artère radiale.

Ce dispositif comprend un cathéter 1 introduit dans l'artère radiale du patient.

Ce cathéter présente deux extrémités :

- l'extrémité intra-artérielle où s'applique le signal hydraulique de la pression artérielle du patient, dit «signal incident».
- l'extrémité extracorporelle qui est reliée par une tubulure 2 à un capteur de pression 3.

La tubulure 2 est relativement longue pour permettre de déplacer le patient, par exemple pour des soins, sa toilette, etc. sans déplacer le capteur de pression 3.

Ainsi, la tubulure 2 présente typiquement une longueur d'environ 1,5 mètre mais peut être plus courte ou plus longue.

Le capteur 3 est par ailleurs relié à une poche 4 de sérum physiologique sous pression au travers d'une résistance importante, qui permet d'assurer une perfusion lente de sérum afin d'éviter un reflux de sang en provenance du cathéter qui provoquerait la formation de caillots.

La liaison hydraulique constituée de la tubulure 2 remplie de sérum physiologique, lequel est théoriquement incompressible, transmet donc - moyennant éventuellement certaines distorsions - la pression artérielle du patient au capteur 3, qui transforme le signal transmis en un signal électrique dit « signal mesuré ».

La résistance s'opposant au passage du sérum physiologique de la poche 4 peut être levée par une action sur une tirette 5, permettant un rinçage et une purge (ou « fast flush » selon la terminologie anglo-saxonne) du circuit par un débit important de sérum.

D'autre part, un robinet 6 à trois voies est intercalé dans la tubulure 2.

Ledit robinet 6 permet, à l'aide d'une seringue 7, d'effectuer des prélèvements sanguins.

Le capteur de pression 3 fait par ailleurs partie d'une chaîne de mesure qui va maintenant être décrite succinctement.

Le capteur de pression 3 est généralement du type piézorésistif et est exploité dans un montage en pont de Wheatstone.

Le capteur 3 est connecté, par l'intermédiaire d'un câble 8, à un module préamplificateur 9 qui assure son alimentation.

Le module préamplificateur 9 comprend un étage d'isolation galvanique (optoélectronique ou autre) pour assurer la protection électrique du patient.

Le module 9 comprend également un dispositif de protection des composants électroniques contre des surcharges éventuelles provoquées par l'utilisation d'un défibrillateur.

Il comprend en outre un filtre passe-bas dont la fréquence de coupure est variable voire réglable entre 5 Hz et 40 Hz.

Enfin, le module préamplificateur 9 assure la numérisation du signal de pression artérielle mesuré pour le rendre exploitable par un moniteur 10 placé au chevet du patient.

Le moniteur 10 affiche le signal numérisé sous la forme d'une courbe de pression artérielle et la transmet à un système central où elle est enregistrée.

Il affiche aussi les valeurs systoliques, diastoliques, et moyennes de cette courbe de pression artérielle et les transmet à un système central où elles sont enregistrées.

En raison des contraintes de stérilité de ces mesures invasives, le capteur 3 est généralement jetable et fourni dans un emballage stérile avec la tubulure 2.

Les avantages de ce type de dispositif sont les suivants.

D'une part, il permet de réaliser une mesure en continu de la pression artérielle, contrairement à la sphygmomanométrie et à l'oscillométrie.

Ceci présente un grand intérêt pour surveiller des patients en instabilité cardiovasculaire.

Par ailleurs, le suivi en continu de la pression artérielle systolique (et non uniquement de la pression artérielle moyenne) se révèle être une information particulièrement intéressante dans trois domaines qui utilisent des indices dérivés de la pression artérielle systolique.

Un premier exemple est celui de l'évaluation du volume sanguin fonctionnel d'un patient sous ventilation mécanique.

En effet, la ventilation mécanique engendre une variation des remplissages ventriculaires droit et gauche, du débit cardiaque et de la différence entre pression artérielle systolique et pression artérielle diastolique.

L'indice désigné par ΔPP est défini au cours d'un même cycle respiratoire comme étant le ratio entre, d'une part, la différence entre l'écart maximum et l'écart minimum entre pression artérielle systolique et pression artérielle diastolique, et d'autre part, la moyenne de ces deux écarts [1].

Un deuxième exemple d'indice dérivé est celui de la mesure du débit cardiaque en continu par analyse du contour de la pression artérielle [2].

Un troisième exemple d'indice dérivé est celui de l'évaluation de la contractilité ventriculaire, pour laquelle on mesure la pente d'ascension maximum du pic systolique ($dP/dt_{max}$) dans les artères [3].

Un autre avantage de ce dispositif est qu'il facilite les nombreux prélèvements sanguins et gazeux du sang artériel, le cathéter étant installé en permanence dans l'artère du patient.

Par ailleurs, la méthode de mesure est robuste, c'est-à-dire qu'elle est peu sensible aux fréquents déplacements du patient, contrairement à la tonométrie artérielle et à la photopléthysmographie digitale.

Enfin, le coût du dispositif est faible à l'achat et à l'entretien (le capteur et la tubulure étant fournis stériles et jetables).

Malgré ces avantages, cette méthode de mesure souffre de plusieurs inconvénients ou limitations.

D'une part, la liaison hydraulique entre le cathéter et le capteur est susceptible d'engendrer des distorsions du signal mesuré par le capteur.

De nombreuses publications scientifiques et techniques établissent la fréquence et la cause de telles distorsions [4] [5].

En effet, la liaison hydraulique, qui est constituée de la tubulure présentant une certaine compliance et contenant une colonne de sérum physiologique, se comporte comme un système mécanique masse/ressort.

Ce système est régi par une équation différentielle du second ordre et peut être caractérisé par une fonction de transfert H(p) du type :

$$H(p) = \frac{1}{\dfrac{p^2}{\omega_0^2} + \dfrac{2z}{\omega_0}p + 1}$$

où p est une variable de Laplace, z est l'amortissement et $\omega_0$ est la pulsation propre, exprimée en rad/s et égale à $2\pi f_0$, où $f_0$ est la fréquence propre, exprimée en Hz.

Selon les valeurs des paramètres de cette fonction de transfert ($f_0$ et z), des distorsions plus ou moins importantes peuvent se produire entre, d'une part le signal incident appliqué à l'extrémité intra-artérielle du cathéter correspondant à la pression artérielle du patient (à l'entrée du système), en fonction de son contenu spectral, et d'autre part le signal mesuré par le capteur (à la sortie du système).

Les distorsions les plus notables peuvent atteindre 20% de la pression artérielle systolique.

Ces distorsions comprennent d'une part une surestimation de la pression artérielle systolique, et d'autre part une sous-estimation de la pression artérielle diastolique, qui apparaissent plus fréquemment sur des signaux à front de montée rapide transmis par une tubulure résonante présentant un faible coefficient d'amortissement.

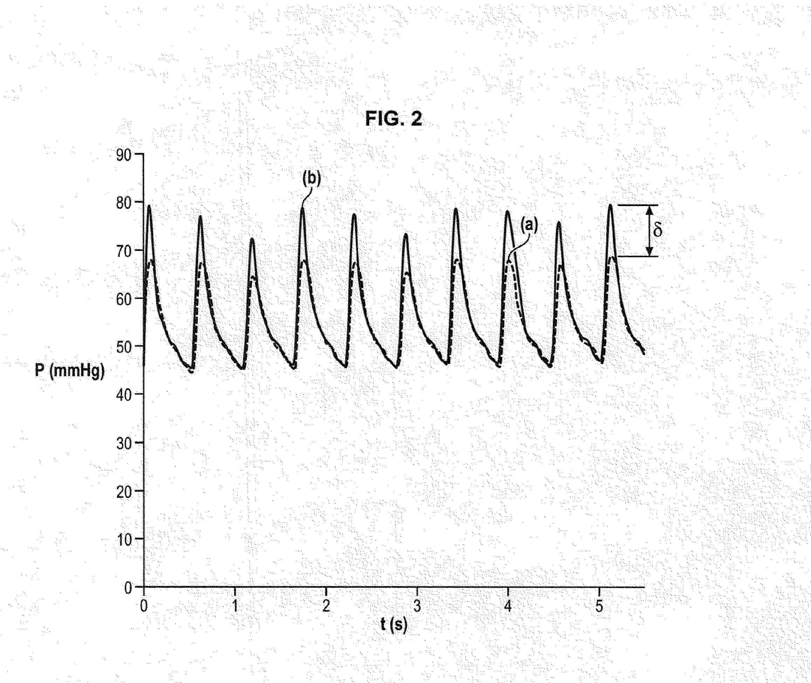

La figure 2 met en évidence, grâce à un tracé de référence mesuré par un capteur de pression situé à l'extrémité intra-artérielle du cathéter (courbe (a)) et un tracé mesuré par un capteur de pression placé, de manière conventionnelle, à une distance d'environ 1,5 m du cathéter (courbe (b)), une surestimation a de la pression artérielle systolique de l'ordre de 15% par le capteur distant.

Par ailleurs, il a été montré que les paramètres ($f_0$, z) de la fonction de transfert pouvaient évoluer de manière significative au fil du temps, probablement en raison de la présence de microbulles dans la tubulure [5].

D'autre part, l'obstruction partielle du cathéter par un caillot peut entraîner une atténuation des pulsations artérielles et une diminution à la fois de la pression artérielle systolique, de la pression artérielle diastolique et de la pression artérielle moyenne.

Outre l'effet direct de ces défauts sur les valeurs des pressions artérielles systolique et diastolique, un impact sur les valeurs des indices dérivés mentionnés ci-dessus peut également être observé.

Or, le personnel soignant détecte plus facilement un amortissement ou une atténuation qu'une résonance sur le signal affiché sur le moniteur.

Dans le premier cas, il effectue habituellement une ou plusieurs purges de la tubulure jusqu'à la disparition du défaut.

Pour pallier le phénomène de résonance, différents dispositifs, recommandations d'emploi et/ou méthodes de test ont été proposés.

Ainsi, le dispositif Accudynamic [6], désormais abandonné, permettait d'ajuster manuellement le facteur d'amortissement.

Le dispositif R.O.S.E. [7] permet, pour une configuration de mesure donnée, de corriger de manière fixe les paramètres dynamiques de l'ensemble de mesure en ajoutant un amortissement.

Le test Gabarith [8] permet de valider l'ensemble d'une chaîne de mesure, incluant le moniteur, par sa réponse dynamique à l'état neuf.

Cependant, ces dispositifs ne tiennent pas compte du fait que les paramètres de la liaison hydraulique évoluent dans le temps.

D'autre part, il est difficile pour les médecins et le personnel soignant d'identifier une altération du signal apparaissant de manière inopinée et pendant une durée aléatoire.

Des études de recherche clinique font état d'un capteur situé au plus près du cathéter pour minimiser les distorsions du signal incident dues à une résonance de la liaison hydraulique.

Cependant, ces recommandations sont difficiles à mettre en oeuvre en pratique courante, car les artéfacts liés aux mouvements du patient sont plus importants que dans le cas où le capteur est distant d'environ 1,5 m du cathéter.

De plus, cette disposition est moins commode pour le personnel soignant, en raison de l'immobilisation du patient qu'elle implique.

Des études ont par ailleurs montré l'impact des caractéristiques mécaniques du matériau de la tubulure, notamment les modules d'Young statiques et dynamiques, sur les distorsions du signal engendrées.

Cependant, même si les fabricants choisissent un matériau particulièrement approprié, ceci permet d'améliorer le comportement dynamique de la liaison hydraulique mais pas d'éliminer les distorsions.

Par ailleurs, un filtrage passe-bas habituellement implémenté sur les moniteurs pourrait réduire dans une certaine mesure les surestimations dues à des résonances.

En effet, les moniteurs des services de réanimation sont généralement programmés avec un filtre passe-bas de 12 Hz, qui permet de fournir une courbe de pression artérielle plus lisse et comportant moins de bruit.

En outre, si la fréquence de résonance de la liaison hydraulique est aux environs de 14 Hz, ce qui est souvent le cas avec des tubulures neuves et/ou exemptes de bulles, le filtrage à 12 Hz permet effectivement de réduire les surestimations dues à des résonances.

Cependant, ce filtrage est opéré au détriment des composantes naturelles du signal incident présentant des fréquences élevées, qui sont éliminées.

Or, on estime généralement qu'il est nécessaire de prendre en compte de 5 à 10 harmoniques de la fréquence fondamentale du signal (qui est de l'ordre de 0,5 à 4 Hz) pour le décrire correctement, certains traitements spécifiques pouvant nécessiter jusqu'à 20 harmoniques [9].

Le filtrage à 12 Hz peut donc conduire à un appauvrissement du signal.

D'autre part, le filtrage à 12 Hz ne permet pas d'éviter les distorsions provoquées par des résonances de fréquence inférieure.

Or, en cas d'accumulation de bulles dans la tubulure, la fréquence de résonance peut descendre jusqu'à 5 ou 6 Hz, sur laquelle le filtrage à 12 Hz n'a pas d'influence.

Enfin, certains moniteurs du marché sont équipés d'une fonction permettant de corriger le signal mesuré en fonction des caractéristiques dynamiques de la liaison hydraulique, qui doivent être paramétrées dans le moniteur par un opérateur.

Cependant, le personnel soignant n'est habituellement pas formé à ce type de réglage et, si des paramètres erronés sont fournis au moniteur, ils risquent d'engendrer des distorsions supplémentaires.

D'autre part, la correction ainsi procurée n'est effective que pendant un bref laps de temps, puisque les caractéristiques de la tubulure évoluent au cours du temps.

Le document DE 39 27 990 [10] envisage un procédé de correction du signal mesuré, consistant à appliquer une impulsion de pression sur la paroi externe de la tubulure et à mesurer la superposition du signal de pression artérielle incident et du signal d'oscillation amortie généré par ladite impulsion.

Un traitement du signal superposé permet de définir les paramètres de correction du signal mesuré.

Cependant, ce signal superposé est complexe et l'extraction des paramètres de correction est donc délicate.

Par ailleurs, une impulsion de pression est peu à même de définir l'amortissement de la liaison.

En outre, ce procédé ne tient pas compte d'artéfacts susceptibles d'intervenir au cours de la mesure ni du fait que la tubulure puisse contenir, par la suite, des bulles qui feront évoluer dans le temps la correction nécessaire.

Or, l'impulsion n'est générée que par une action du personnel soignant et, même si le signal mesuré est correctement corrigé dans un bref laps de temps suivant l'application de l'impulsion, rien ne garantit que cette correction reste longtemps effective. D'autres exemples des documents envisageant la correction des mesures de pressions artérielle utilisant ce même système et essayant de quantifier les distorsions du signal lors de sa correction sont US2006/064021 A1 ou US2010/094113 A1.

Il subsiste donc un besoin d'améliorer la qualité d'un signal de pression artérielle mesuré par le capteur.

Un but de l'invention est donc de procurer un procédé et un dispositif qui permettent d'améliorer, sur toute la durée de vie de la liaison hydraulique, la qualité du signal mesuré et la fiabilité des informations fournies au personnel soignant.

Compte tenu des habitudes prises de longue date par le personnel hospitalier avec le dispositif conventionnel de mesure de la pression artérielle par cathétérisme artériel, il est souhaitable de modifier aussi peu que possible le dispositif existant.

Naturellement, le dispositif devra répondre pleinement aux exigences en termes de stérilité et sécurité à l'égard du patient qui sont requises pour ce type d'équipement.

## BREVE DESCRIPTION DE L'INVENTION

Conformément à l'invention, défini dans la revendication 1, il est proposé un procédé de surveillance et, le cas échéant, de correction en continu de la mesure de la pression artérielle effectuée par un capteur de pression relié, par l'intermédiaire d'une liaison hydraulique, à un cathéter préalablement introduit dans l'artère d'un patient, ladite liaison hydraulique transmettant au capteur un signal de pression artérielle du patient, dit « signal incident » appliqué à l'extrémité intra-artérielle du cathéter et le capteur transformant le signal transmis par la liaison hydraulique en un signal électrique, dit « signal mesuré ».

Ledit procédé est caractérisé en ce qu'il comprend des étapes de :

- détermination, à partir du signal mesuré, des paramètres dynamiques de ladite liaison hydraulique, lesdits paramètres comprenant la fréquence propre et le coefficient d'amortissement de la liaison hydraulique,
- analyse du contenu fréquentiel du signal incident, de manière à déterminer la composante de fréquence maximale dudit signal,
- détection, à partir desdits paramètres dynamiques de la liaison hydraulique et, le cas échéant, de la fréquence maximale à transmettre, de l'une des situations suivantes :

  (a) l'aptitude de la liaison hydraulique à transmettre le signal incident au capteur avec une distorsion inférieure à un seuil,
  (b) l'aptitude de la liaison hydraulique à transmettre le signal incident au capteur avec une distorsion supérieure audit seuil et la possibilité de corriger le signal mesuré,
  (c) l'inaptitude de la liaison hydraulique à transmettre le signal incident au capteur avec une distorsion inférieure audit seuil et l'impossibilité de corriger le signal mesuré,

ledit procédé comprenant en outre l'application périodique d'une action mécanique sur la paroi externe de la tubulure qui assure la liaison hydraulique.

La mise en oeuvre de ce procédé n'inclut pas la mise en place du cathéter, qui a été installé préalablement dans l'artère du patient.

De manière particulièrement avantageuse, ladite action mécanique comprend une percussion de la paroi externe de la tubulure qui assure la liaison hydraulique.

De manière alternative, ladite action mécanique comprend un train de sollicitations sinusoïdales de fréquence variable appliqué à la paroi externe de la tubulure qui assure la liaison hydraulique.

De préférence, ladite action mécanique est synchronisée avec une diastole ou segmentée de manière synchronisée avec plusieurs diastoles successives.

La détermination des paramètres dynamiques de la liaison hydraulique peut être réalisée à partir de l'analyse du signal mesuré en réponse à une purge et/ou à ladite action mécanique appliquée à la paroi externe de la tubulure qui assure la liaison hydraulique et/ou à partir de l'analyse du contenu fréquentiel du signal.

Selon un mode de réalisation, la détection de l'une des situations (a), (b) ou (c) comprend une comparaison du signal mesuré et du signal mesuré corrigé en fonction des paramètres dynamiques de la liaison hydraulique.

Selon un autre mode de réalisation, la détection de l'une des situations (a), (b) ou (c) comprend le positionnement, sur un abaque fréquence propre-amortissement, du point représentatif des paramètres dynamiques de la liaison hydraulique par rapport à une aire de tolérance déterminée par la composante de fréquence maximale du signal utilisé.

Dans la situation (b), le procédé comprend avantageusement la correction du signal mesuré en fonction des paramètres dynamiques de la liaison hydraulique.

Dans la situation (c), le procédé comprend l'émission d'un signal d'alerte, notamment lumineux.

De préférence, ledit signal d'alerte comprend une indication d'une action à réaliser par le personnel soignant, telle qu'une purge ou le remplacement de la liaison hydraulique et/ou du cathéter.

Par ailleurs, le procédé peut comprendre l'enregistrement du signal mesuré, le cas échéant corrigé.

Le procédé peut également comprendre la transmission du signal mesuré, le cas échéant corrigé, à un système de traitement.

De préférence, si l'analyse du contenu fréquentiel du signal incident détecte un glissement spectral et/ou une variation du poids relatif des composantes de fréquences supérieures, on met en oeuvre ladite étape de détermination des paramètres dynamiques de la liaison hydraulique de manière à réactualiser lesdits paramètres.

De manière optionnelle mais avantageuse, le procédé comprend en outre la détection d'un découplage entre la pression artérielle centrale et la pression artérielle radiale du patient, ladite détection comprenant la surveillance du rapport entre les harmoniques supérieures et les harmoniques inférieures du signal mesuré et, si ledit rapport diminue brutalement :

- la mise en oeuvre d'une mesure tonométrique humérale et/ou carotidienne,
- la détermination du rapport entre les harmoniques supérieures et les harmoniques inférieures du signal de pression humérale et/ou carotidienne,
- la comparaison dudit rapport avec le rapport entre les harmoniques supérieures et les harmoniques inférieures du signal mesuré.

De manière alternative ou complémentaire, le procédé peut en outre comprendre la détection d'un découplage entre la pression artérielle centrale et la pression artérielle radiale du patient, ladite détection comprenant la mise en oeuvre d'une mesure tonométrique de l'artère humérale/brachiale et/ou carotidienne, la surveillance de la vitesse de propagation de l'onde de pouls du patient déterminée à partir de ladite mesure tonométrique et du signal mesuré, et la comparaison de ladite vitesse avec une valeur de référence.

En cas d'impossibilité d'effectuer une mesure tonométrique carotidienne, on évalue la pression aortique par une fonction de transfert à partir de la mesure de la pression humérale.

De préférence, on met en oeuvre chacune des deux étapes de détection d'un découplage décrites ci-dessus ; si chacune des deux détections met en évidence un découplage de la pression artérielle centrale et de la pression artérielle radiale du patient, une alarme est alors émise.

Un autre objet de l'invention est défini dans la revendication 10 associée et concerne un dispositif de surveillance et, le cas échéant, de correction en continu de la mesure de la pression artérielle effectuée par un capteur de pression relié, par l'intermédiaire d'une liaison hydraulique, à un cathéter préalablement introduit dans l'artère d'un patient, ladite liaison hydraulique transmettant au capteur un signal de pression artérielle du patient, dit « signal incident » appliqué à l'extrémité intra-artérielle du cathéter, et le capteur transformant le signal transmis par la liaison hydraulique en un signal électrique, dit « signal mesuré ».

Ledit dispositif est caractérisé en ce qu'il comprend :

- un module de surveillance et de correction adapté pour mettre en oeuvre les étapes consistant à :

    - déterminer, à partir du signal mesuré, les paramètres dynamiques de ladite liaison hydraulique, lesdits paramètres comprenant la fréquence propre et le coefficient d'amortissement de la liaison hydraulique,
    - analyser le contenu fréquentiel du signal incident, de manière à déterminer la composante de fréquence maximale à transmettre dudit signal,
    - détecter, à partir desdits paramètres dynamiques de la liaison hydraulique, et, le cas échéant, de la fréquence maximale à transmettre, l'une des situations suivantes :

        (a) l'aptitude de la liaison hydraulique à transmettre le signal incident au capteur avec une distorsion inférieure à un seuil,
        (b) l'aptitude de la liaison hydraulique à transmettre le signal incident au capteur avec une distorsion supérieure audit seuil et l'aptitude dudit module à corriger le signal mesuré,
        (c) l'inaptitude de la liaison hydraulique à transmettre le signal incident au capteur avec une distorsion inférieure audit seuil et l'inaptitude dudit module à corriger le signal mesuré,

- un module actuateur, adapté de sorte à appliquer de manière périodique une action mécanique sur la paroi externe de la tubulure qui assure la liaison hydraulique.

Ledit dispositif s'intègre dans une chaîne de mesure de pression artérielle conventionnelle comprenant ledit cathéter, ladite liaison hydraulique et ledit capteur de pression.

L'avantage d'un tel dispositif est de conserver la disposition conventionnelle adoptée en clinique (c'est-à-dire avec capteur déporté du cathéter) avec ses avantages reconnus, notamment le fait que le capteur est moins sensible aux artéfacts de mouvement du patient, ainsi que le maintien d'une meilleure mobilité du patient.

D'autre part, ledit dispositif remédie aux inconvénients de la disposition conventionnelle, en détectant et, le cas échéant, en corrigeant les distorsions dues aux caractéristiques dynamiques de la liaison hydraulique.

Le dispositif assure également un suivi permanent des incidents et de la qualité de la liaison, permettant au clinicien de savoir s'il peut se fier à son matériel de mesure de pression artérielle.

Très facile à mettre en place, le dispositif ne nécessite, par défaut, aucun réglage de la part de l'opérateur, et reste ensuite totalement transparent pour le personnel hospitalier.

Sa neutralisation complète, temporaire ou pour le reste de la durée de vie de la liaison peut être réalisée instantanément, par simple pression sur un bouton.

Selon une forme d'exécution préférée, le module de surveillance et de correction comprend :

- un premier processeur, adapté pour déterminer les paramètres dynamiques de la liaison hydraulique, analyser le contenu fréquentiel du signal incident, détecter l'une des situations (a), (b) ou (c) et, le cas échéant, définir des paramètres de correction du signal mesuré en fonction des paramètres dynamiques de la liaison hydraulique,
- un second processeur, adapté pour, le cas échéant, corriger en continu le signal mesuré à partir des paramètres de correction définis par le premier processeur.

De manière particulièrement avantageuse, lesdits premier et second processeurs sont connectés de sorte à assurer des traitements redondants, ce qui renforce la sécurité du dispositif.

Le module actuateur comprend de préférence un actionneur piézoélectrique ou un dispositif électromécanique.

Selon un mode de réalisation, le dispositif comprend une liaison filaire entre le module de surveillance et de correction et le module actuateur, ladite liaison filaire étant adaptée pour fournir au module actuateur un signal de synchronisation avec une diastole et/ou de l'énergie électrique en provenance du module de surveillance.

Selon un autre mode de réalisation, le dispositif comprend une liaison sans fil entre le module de surveillance et le module actuateur, ladite liaison sans fil étant adaptée pour fournir au module actuateur un signal de synchronisation avec une diastole émis par le module de surveillance.

Dans ce cas, le module actuateur comprend avantageusement une batterie rechargeable pour son alimentation en énergie.

Le module de surveillance et de correction peut être inclus dans un boîtier connectable entre le capteur de pression et un moniteur affichant le signal mesuré.

Selon une forme d'exécution particulière, le module actuateur est inclus dans le même boîtier que le module de surveillance.

De manière alternative, le module actuateur est adapté pour être rendu solidaire de la tubulure assurant la liaison hydraulique, à distance du module de surveillance et de correction.

De manière particulièrement avantageuse, le dispositif comprend en outre un système de traitement adapté pour communiquer avec le module de surveillance.

Ledit système de traitement peut être adapté pour traiter le signal mesuré, le cas échéant corrigé, transmis par le module de surveillance.

Ledit système de traitement peut en outre être adapté pour transmettre au module de surveillance des consignes de modification du fonctionnement dudit module de surveillance.

Ledit système de traitement peut être inclus dans un assistant personnel portable.

Ledit dispositif peut en outre comprendre un boîtier de mesure tonométrique pour permettre la détection d'un éventuel découplage entre la pression artérielle centrale et la pression artérielle périphérique.

## BREVE DESCRIPTION DES DESSINS

D'autres caractéristiques et avantages de l'invention ressortiront de la description détaillée qui va suivre, en référence aux dessins annexés sur lesquels :

- la figure 1 est un schéma d'un dispositif conventionnel de mesure de la pression artérielle par cathétérisme de l'artère radiale,
- la figure 2 présente un tracé de la pression artérielle obtenu par un dispositif du type de celui illustré à la figure 1 et un tracé de référence obtenu par un capteur situé au niveau de l'extrémité intra-artérielle du cathéter,
- la figure 3 est un schéma d'un dispositif de mesure de la pression artérielle par cathétérisme de l'artère radiale incorporant un dispositif de surveillance et de correction selon un mode de réalisation de l'invention,
- la figure 4 est un schéma illustrant l'implantation du module de surveillance et de correction dans la chaîne de mesure existante,
- la figure 5 illustre de manière schématique l'architecture du module de surveillance et de correction,
- la figure 6 est un schéma d'un dispositif de mesure de la pression artérielle par cathétérisme de l'artère radiale incorporant un dispositif de surveillance et de correction selon un autre mode de réalisation de l'invention,
- la figure 7 illustre la courbe de réponse à une consigne en forme d'échelon, employée dans une méthode dite percussionnelle de détermination des paramètres dynamiques de la liaison hydraulique,
- la figure 8 illustre une courbe de réponse à un train de sollicitations mécaniques sinusoïdales de fréquence variable, employée dans une méthode dite harmonique de détermination des paramètres dynamiques de la liaison hydraulique,
- la figure 9 illustre le tracé du spectre fréquentiel d'un signal incident, permettant de déterminer la fréquence fondamentale dudit signal (ff) et la composante de fréquence maximale à transmettre (fh),
- la figure 10 présente les tracés d'un signal mesuré brut et d'un signal mesuré corrigé, dont la comparaison permet de déterminer la surestimation de la pression systolique introduite par une liaison hydraulique résonnante,
- la figure 11 illustre la mise en oeuvre d'une méthode d'estimation de l'aptitude de la liaison hydraulique à reproduire le signal incident avec une distorsion inférieure à une valeur donnée,
- les figures 12A et 12B illustrent la variation de la pression artérielle aortique (courbe (a)), radiale (courbe (b)) et fémorale (courbe (c)), respectivement dans une situation normale et dans une situation de choc septique,
- les figures 13A et 13B illustrent respectivement les pressions aortique et radiale chez un porc normal et chez un porc en situation de choc septique,
- la figure 14 illustre respectivement la distribution de la pression artérielle de l'aorte vers l'artère radiale chez l'un de ces patients avant (courbe du haut) et après (courbe du bas) la mise en oeuvre d'une circulation extracorporelle, en fonction de la distance par rapport à l'artère radiale,
- la figure 15 illustre la vitesse moyenne de propagation de l'onde de pouls en fonction de la distance par rapport à l'artère radiale, avant (courbe (a)) et après (courbe (b)) la mise en oeuvre d'une circulation extracorporelle chez des patients présentant un gradient de pression entre la pression artérielle centrale et la pression artérielle périphérique.

## DESCRIPTION DETAILLEE DE L'INVENTION

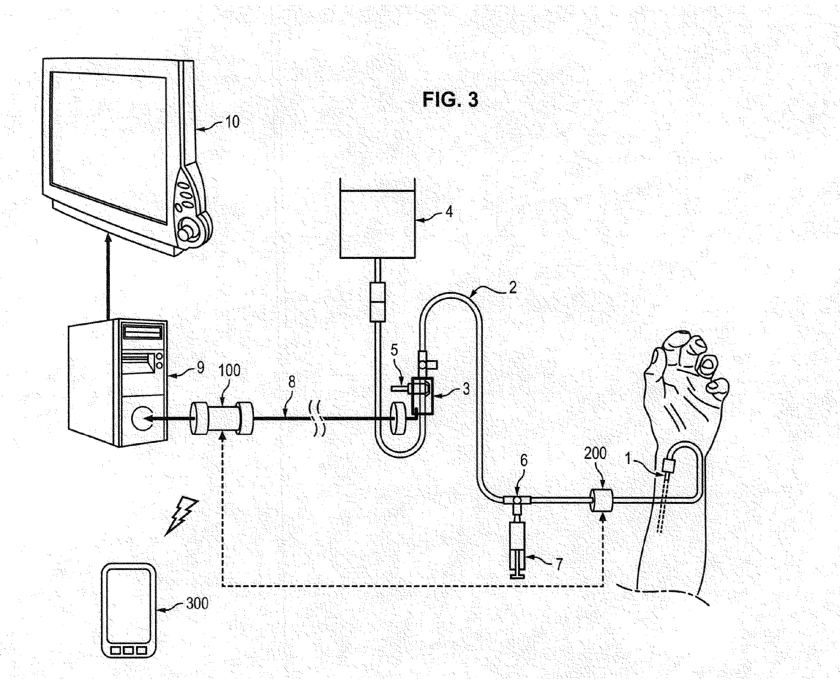

La figure 3 est un schéma d'un mode de réalisation d'un dispositif de mesure de la pression artérielle par cathétérisme de l'artère radiale dans lequel est inséré le dispositif de surveillance et, le cas échéant, de correction, mentionné plus haut.

Les composants remplissant la même fonction que ceux déjà décrits en référence à la figure 1 reprennent les mêmes signes de référence.

Le dispositif de surveillance et de correction comprend principalement un module de surveillance et de correction 100 et un module actuateur 200, qui sont décrits successivement ci-après.

Module de surveillance et de correction

*Fonctions remplies par le module de surveillance et de correction*

Le module de surveillance et de correction 100 comprend des composants électroniques et d'informatique embarquée destinés à remplir en temps réel les fonctions suivantes, qui permettent de réaliser la surveillance et, le cas échéant, la correction de la mesure de pression artérielle.

Une fonction consiste à caractériser les paramètres dynamiques intrinsèques de la liaison hydraulique 2, à savoir sa fréquence propre $f_0$ et son coefficient d'amortissement z.

On décrira plus bas différentes méthodes, dont certaines font appel au module actuateur 200, qui permettent cette caractérisation.

Une autre fonction comprend l'analyse fréquentielle du signal de pression artérielle incident, de manière à déterminer la composante de fréquence maximum (notée fh) que la liaison hydraulique doit transmettre correctement pour fournir au personnel soignant une représentation fidèle de la pression artérielle du patient.

Une autre fonction comprend une estimation, à partir des paramètres ($f_0$, z et éventuellement fh) déterminés précédemment, de l'aptitude de la liaison hydraulique à reproduire le signal incident avec une plage de distorsion donnée.

Plus précisément, cette phase d'estimation permet de détecter l'une des trois situations suivantes :

(a) une situation dans laquelle la liaison hydraulique est apte à transmettre le signal incident avec une distorsion inférieure à un seuil de tolérance déterminé, par exemple une distorsion inférieure à 5% (on considère que dans ce cas aucune correction du signal mesuré n'est nécessaire) ;

(b) une situation dans laquelle la liaison hydraulique transmet le signal incident avec une distorsion supérieure audit seuil de tolérance, mais avec une possibilité, pour le module de surveillance, de corriger le signal mesuré ;

(c) une situation dans laquelle la liaison hydraulique transmet le signal incident avec une distorsion supérieure audit seuil de tolérance, mais sans possibilité, pour le module de surveillance, de corriger le signal mesuré.

Une autre fonction susceptible d'être mise en oeuvre par le module de surveillance 100 est la correction automatique du signal mesuré, lorsqu'elle est possible (c'est-à-dire essentiellement dans la situation (b)), en utilisant les paramètres dynamiques de la liaison hydraulique déterminés dans la phase de caractérisation.

Une autre fonction comprend la détection des artéfacts de mesure et des incidents divers pouvant intervenir sur la liaison hydraulique et sur le cathéter, et de nature à remettre en cause la pertinence des informations données par le moniteur.

Une autre fonction comprend la fourniture au personnel soignant d'une information sur l'état actuel de la liaison hydraulique et sur la nécessité éventuelle d'une action correctrice de sa part (typiquement, une purge ou le remplacement de la tubulure et/ou du cathéter).

Cette information est fournie par l'intermédiaire d'une interface homme-machine simplifiée, pouvant par exemple consister en un voyant lumineux pouvant adopter une couleur différente en fonction de chacune des situations (a), (b) et (c).

De manière avantageuse, le personnel soignant peut choisir l'un des trois modes de fonctionnement suivants du module de surveillance et de correction :

- un mode dit « actif », qui est par exemple le mode par défaut, dans lequel le module de surveillance et de correction analyse le signal incident et les paramètres dynamiques de la liaison hydraulique, évalue la capacité de la liaison à reproduire fidèlement le signal incident, corrige éventuellement le signal mesuré et alerte en cas de nécessité d'une intervention du personnel soignant ;
- un mode dit « passif », dans lequel le module de surveillance et de correction fonctionne comme dans le mode actif, à l'exception du fait qu'il ne modifie aucunement le signal mesuré (notamment, il n'effectue aucune correction, même lorsque celle-ci serait nécessaire et réalisable) ; seule la fonction de surveillance est alors active ;
- un mode dit « neutre », dans lequel le module de surveillance est totalement désactivé.

*Architecture du module de surveillance et de correction*

De manière particulièrement avantageuse, le module de surveillance et de correction 100 comprend deux processeurs aptes à communiquer entre eux et qui assurent chacun une partie des fonctions exposées ci-dessus.

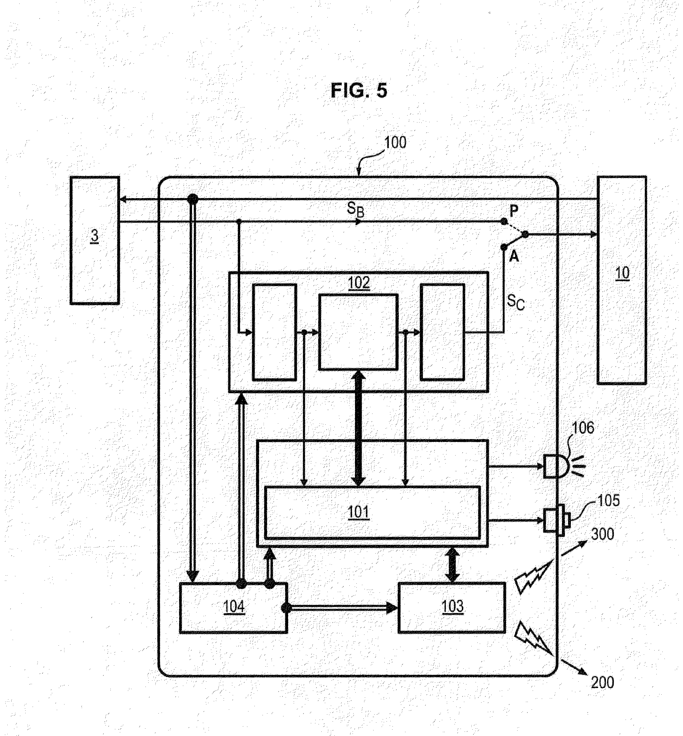

Cette architecture est illustrée de manière schématique à la figure 5.

Le module 100 est intercalé entre le capteur 3 et le pré-amplificateur 9 du moniteur 10.

Le capteur 3 transmet au module 100 un signal mesuré brut, noté $S_B$.

L'alternative entre les modes passif et actif est représentée schématiquement par la commutation entre les repères P et A.

Un premier processeur 101 réalise la majeure partie du traitement du signal mesuré.

Ce premier processeur 101 est ainsi configuré pour caractériser les paramètres dynamiques de la liaison hydraulique.

A cet égard, le premier processeur 101 analyse la réponse de la liaison hydraulique aux purges qui sont déclenchées par le personnel soignant selon le protocole en vigueur dans le service et/ou aux actions mécaniques sollicitant la tubulure, exercées périodiquement par le module actuateur 200.

Le premier processeur 101 réalise en outre l'analyse fréquentielle du signal incident et son évolution dans le temps, notamment pour déterminer la fréquence fondamentale ff et les autres composantes significatives (apportant une contribution à l'analyse) dudit signal.

A partir de ces analyses, le premier processeur 101 estime ensuite l'aptitude de la liaison hydraulique à reproduire fidèlement le signal incident dans une plage de tolérance donnée.

En fonction de la situation (a), (b) ou (c), le premier processeur 101 définit l'éventuelle correction à apporter au signal mesuré.

Ainsi, dans la situation (a), le premier processeur 101 fournit au moniteur le signal brut, exempt de correction.

Dans la situation (b), le premier processeur 101 détermine les nouveaux paramètres de correction à appliquer et les actualise dans un second processeur 102 qui, comme on le verra plus bas, est destiné à la correction du signal mesuré.

Enfin, dans la situation (c), le premier processeur 101 délivre une alerte destinée au personnel soignant, par l'intermédiaire de l'interface homme-machine simplifiée évoquée plus haut.

Par défaut, il fournit dans ce cas au moniteur la version brute du signal.

Ledit premier processeur 101 réalise également une détection précoce de la possible dérive des paramètres dynamiques de la liaison hydraulique si une variation importante du contenu spectral du signal mesuré est observée (glissement spectral et/ou variation du poids relatif des composantes de fréquences supérieures).

Dans ce cas, une séquence de caractérisation de la liaison pourra être déclenchée par anticipation.

Ledit premier processeur 101 réalise également la détection et le suivi des événements (artéfacts de mouvements, obstructions du cathéter, etc.) survenant au cours de la mesure et les consigne dans un journal qui reste mémorisé au long de la durée de vie de la tubulure.

Le premier processeur 101 communique en outre avec le module actuateur 200, par une liaison filaire ou par une liaison sans fil.

Le cas échéant, le premier processeur 101 communique également, par une liaison sans fil, avec un système de traitement déporté 300.

A cet effet, le module de surveillance et de correction 100 comprend un module de gestion des communications 103, qui utilise par exemple la technologie radiofréquences pour communiquer avec le module actuateur 200 et/ou le système de traitement 300.

Le module de surveillance et de correction comprend un second processeur 102, qui est dédié à la correction en continu du signal mesuré et transmet, en mode actif A, le signal mesuré corrigé $S_C$ au moniteur 10 par l'intermédiaire du préamplificateur 9.

Les paramètres de correction sont déterminés par le premier processeur 101 qui les met à jour périodiquement dans le second processeur 102, de manière à refléter les variations dans le temps des paramètres $f_0$ et z de la liaison hydraulique.

Le second processeur 102 peut également réaliser une partie des fonctions de surveillance de la liaison hydraulique, comme la détection d'artéfacts.

Cependant, si un événement est détecté et nécessite un traitement substantiel, le premier processeur 101 est sollicité.

La consommation énergétique est gérée par un module de gestion d'énergie 104.

On alimente de préférence le module de surveillance et de correction 100 par la tension d'excitation fournie par le moniteur 10 au pont résistif du capteur de pression 3.

Or, cette source présente une capacité limitée, qui nécessite une conception particulièrement économe sur le plan énergétique du module de surveillance et de correction.

Une telle performance peut être atteinte en choisissant judicieusement l'architecture des deux processeurs 101, 102 et les actions qu'ils doivent assurer.

A cet égard, le second processeur 102, qui est constamment en activité, est dédié à des tâches peu consommatrices d'énergie.

Sa technologie est choisie en conséquence (composant analogique programmable par exemple).

Par contraste, le premier processeur 101 est dédié au traitement numérique du signal, ce qui nécessite des travaux intensifs, mais il fonctionne de manière discontinue.

Il est basé par exemple sur une technologie DSP (acronyme du terme anglo-saxon « Digital Signal Processor »).

Le premier processeur 101 n'est sollicité que lors de l'apparition d'événements importants (purges, artéfacts) et/ou sur une base périodique choisie pour l'actualisation de la caractérisation de la liaison hydraulique (à titre purement indicatif, toutes les demi-heures par exemple).

Ainsi, la durée de l'opération et des calculs est limitée par rapport à celle des périodes d'inactivité (le mode de sommeil autorisant une très basse consommation d'énergie).

Par conséquent, la consommation énergétique moyenne du module de surveillance et de correction 100 reste très faible, avec des pics localisés correspondant aux périodes de traitement du signal.

Par ailleurs, le module de surveillance et de correction 100 peut comprendre une réserve d'énergie qui tire parti de la source permanente mais limitée en puissance fournie par l'alimentation du capteur, en chargeant un stockage tampon adapté pour répondre à des besoins en puissance ponctuels.

Ladite source d'énergie peut également servir à alimenter le module actuateur 200, dans certains modes de réalisation qui seront décrits plus bas.

Par ailleurs, les premier et second processeurs 101, 102 sont chacun en mesure de vérifier, d'une part, le fonctionnement effectif de l'autre processeur et, d'autre part, la cohérence de certains résultats fournis de manière redondante par les deux processeurs.

Ceci permet d'assurer la sécurité du module de surveillance et de correction 100.

Naturellement, le module de surveillance et de correction pourra être implémenté avec toute autre architecture que celle décrite ci-dessus, pour autant que les fonctions mentionnées plus haut soient remplies.

Le module de surveillance et de correction est inclus dans un boîtier miniaturisé qui assure la protection des processeurs et qui comprend des connecteurs appropriés pour interconnecter le module au dispositif de mesure existant.

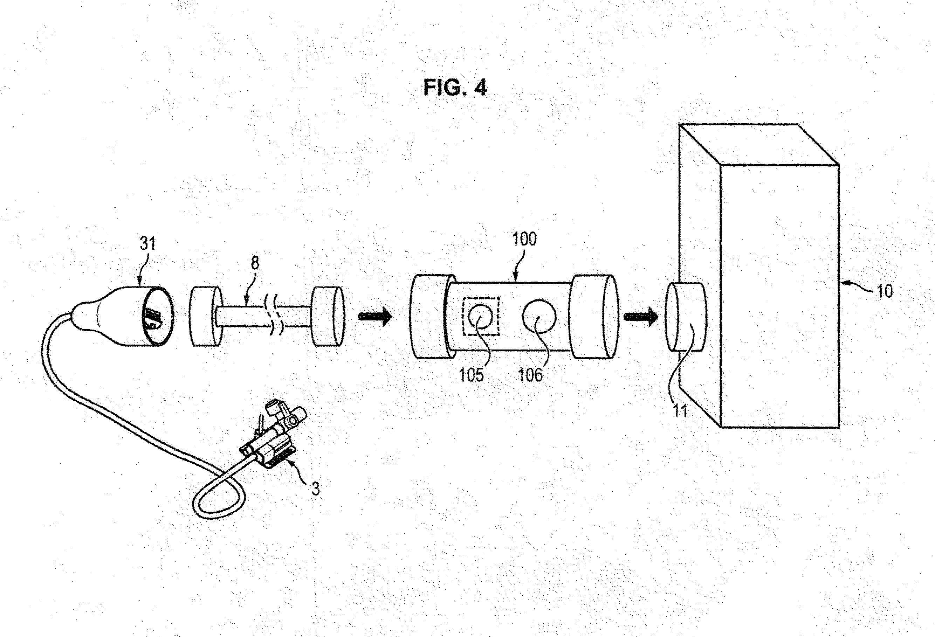

Comme on peut le voir à la figure 4, le module de surveillance et de correction 100 est avantageusement connecté entre le câble 8 branché sur une prise 31 du capteur 3 et la prise 11 du moniteur 10.

Ainsi, le module de surveillance et de correction s'intègre aisément à la chaîne de mesure existante, sans générer d'encombrement supplémentaire.

Ledit boîtier peut en outre incorporer une interface homme-machine simplifiée, comprenant un voyant lumineux 106 susceptible de produire une couleur différente selon la situation (a), (b) ou (c), ainsi qu'un bouton poussoir 105 permettant de sélectionner le mode actif, passif ou neutre.

Par exemple, le voyant lumineux 106 est vert dans la situation (a), orange dans la situation (b) et rouge dans la situation (c).

Ledit module est de préférence conçu de manière à ne pas être jeté après une utilisation chez un patient mais pour être réutilisé.

A cet effet, le boîtier doit pouvoir être stérilisé par les techniques habituellement employées en milieu hospitalier.

## Module actuateur

Le module actuateur 200 est destiné à appliquer une action mécanique à la tubulure de manière à exciter la liaison hydraulique, le signal mesuré étant ensuite analysé par le module de surveillance 100 afin d'en déduire les paramètres dynamiques de la liaison.

De préférence, ladite action mécanique est appliquée à proximité du cathéter 1.

Selon un mode de réalisation, le module actuateur 200 génère une impulsion destinée à fournir une excitation brève en entrée de la liaison hydraulique 2.

Selon un autre mode de réalisation, le module actuateur 200 génère un bref train de sollicitations sinusoïdales de fréquence variable.

De manière plus générale, le module actuateur 200 peut générer toute séquence de sollicitations mécaniques de courte durée et apte à caractériser la liaison hydraulique 2 sans perturber notablement le signal de pression artérielle.

Il est possible de combiner lesdits modes de réalisation et d'exciter la liaison hydraulique avec différents types d'actions mécaniques.

Ainsi, on peut avantageusement utiliser une impulsion pour déterminer la fréquence propre $f_0$ de la liaison (méthode percussionnelle) et un train de sollicitations sinusoïdales de fréquence variable pour déterminer le coefficient d'amortissement z de la liaison hydraulique (méthode harmonique).

Sous l'effet de l'action mécanique du module actuateur 200, la liaison hydraulique 2 fournit une réponse caractéristique d'un système du second ordre et dépendant de ses paramètres dynamiques.

Cette réponse se superpose au signal incident pour fournir un signal complexe qui est mesuré et analysé par le module de surveillance 100, qui en déduit les paramètres dynamiques recherchés.

Selon un mode de réalisation avantageux, on synchronise l'action mécanique sur une diastole, qui correspond à une portion de signal dont le contenu fréquentiel permet une analyse plus facile que pour d'autres segments de ce signal.

A cet effet, le module de surveillance 100 (plus précisément, dans le cas de l'architecture bi-processeur, le premier processeur 101 décrit ci-dessus) envoie au module actuateur 200 un signal de synchronisation qui déclenche l'action mécanique pendant une diastole.

L'envoi de ce signal de synchronisation peut être réalisé soit par une liaison filaire soit par une liaison sans fil.

Eventuellement, selon la durée du train de sollicitations mécaniques, celui-ci pourra être décomposé en plusieurs séquences distribuées sur plusieurs diastoles successives.

Le module actuateur 200 comprend un actionneur agencé de sorte à enserrer la tubulure 2, de manière à réaliser un contact mécanique avec la paroi externe de celle-ci, sans la déformer notablement.

L'action mécanique générée par l'actionneur provoque une variation de pression dans le sérum physiologique contenu dans la tubulure.

Selon un mode de réalisation, l'actionneur est un élément piézoélectrique actif.

Dans ce cas, la source d'énergie électrique applique une tension entre les faces métallisées de la céramique piézoélectrique, qui se déforme en conséquence et transmet, par contact mécanique, une contrainte de déformation à la tubulure.

La consommation électrique moyenne du module actuateur 200 est très faible, celui-ci ne fonctionnant que de manière périodique.

Ainsi, à titre indicatif, on déclenche une action mécanique sur la liaison hydraulique selon une période d'une demi-heure, la durée de ladite action étant de quelques millisecondes (méthode percussionnelle) à quelques secondes (méthode harmonique).

A cet effet, le module actuateur 200 peut comprendre une source d'énergie interne, sous forme d'une batterie, de préférence rechargeable par un dispositif sans contact (induction), ou bien externe, par l'intermédiaire de la liaison filaire avec le module de surveillance 100.

Selon d'autres modes de réalisation, l'actionneur peut être un dispositif électromécanique comprenant une bobine et un noyau plongeur, ou de manière plus générale tout dispositif permettant de traduire un signal électrique en une sollicitation mécanique.

Ledit module 200 est de préférence conçu de manière à ne pas être jeté après une utilisation chez un patient mais pour être réutilisé.

A cet effet, le module actuateur 200 doit pouvoir être monté et démonté facilement de la tubulure 2.

Il doit également pouvoir être stérilisé par les techniques habituellement employées en milieu hospitalier.

Selon un mode de réalisation, le module actuateur 200 est installé à distance du module de surveillance et de correction 100.

Tel est le cas sur la figure 3, où le module actuateur 200 est placé à proximité du cathéter tandis que le module 100 de surveillance et de correction est situé à proximité du capteur de pression 3.

Cette implémentation est avantageuse pour le traitement du signal.

En effet, la distance entre le point d'application des actions mécaniques et le capteur de pression rend plus facile la caractérisation des paramètres dynamiques de la liaison hydraulique.

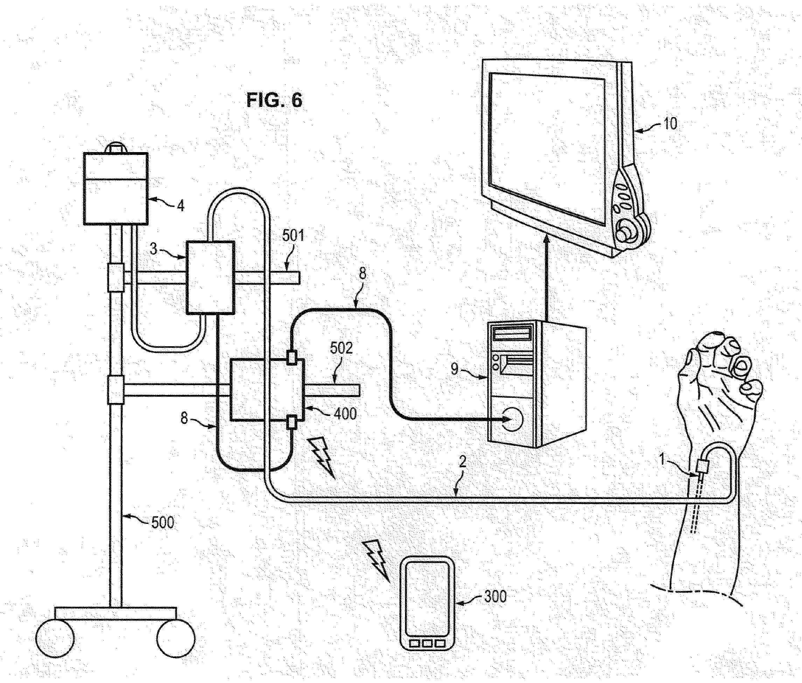

Selon une autre forme d'exécution, illustrée à la figure 6, le module actuateur et le module de surveillance et de correction sont installés dans un même boîtier 400.

Dans ce cas, le boîtier 400 est installé à proximité du capteur de pression 3.

Par exemple, le capteur 3 et le boîtier 400 sont installés sur des rails 501, 502 d'une potence de perfusion 500 qui porte la poche 4 de sérum physiologique.

La tubulure assurant la liaison hydraulique 2 est localement enserrée dans le boîtier 400 de sorte à être en contact avec le module actuateur.

Le boîtier 400 est relié électriquement d'une part au capteur 3 et d'autre part au préamplificateur 9 du moniteur 10 par des câbles électriques 8.

Il est muni à cet effet de prises électriques standard adaptées à ces câbles.

Un avantage de cette implémentation est que l'ensemble du dispositif étant déporté à proximité du capteur, il laisse libre l'extrémité de la tubulure du côté du cathéter 1, qui est une zone sensible vis-à-vis des risques d'infection nosocomiales.

De plus, cette implémentation augmente le confort du patient et du personnel hospitalier qui pourraient éprouver une gêne dans la présence d'un actuateur indépendant placé à proximité de la voie artérielle.

Par ailleurs, le boîtier 400 simplifie l'alimentation du module de surveillance et de correction et du module actuateur en rendant possible, grâce à la proximité des deux modules, une alimentation commune.

De même, le transfert d'informations entre les deux modules est simplifié.

Le boîtier 400 facilite également le passage des câbles électriques reliant le correcteur au capteur et au moniteur.

Enfin, l'interface utilisateur du module de surveillance et de correction comprenant le voyant d'état et le bouton poussoir lumineux de sélection de mode peuvent être installés sur la face avant du boîtier 400, de sorte à se trouver à hauteur de regard du personnel soignant.

Système de traitement déporté

De manière avantageuse mais optionnelle, le dispositif de surveillance peut comprendre en outre un système de traitement déporté 300, par exemple incorporé à un assistant personnel portable.

Ledit système de traitement 300 communique de préférence avec le module de surveillance 100 par une liaison sans fil et permet de réaliser des opérations complémentaires des opérations de surveillance et de contrôle.

Ainsi, le système de traitement 300 peut permettre de traiter le signal mesuré pour en déduire les indices dérivés de la pression artérielle systolique décrits plus haut.

Ledit système peut également permettre d'afficher les tracés de pression artérielle, de les enregistrer en vue d'assurer la traçabilité des mesures et/ou d'effectuer un suivi qualité.

Notamment, il peut permettre d'obtenir une visualisation détaillée de différentes courbes et paramètres sous une forme graphique et/ou numérique, par exemple :

- courbes de pression artérielle brute et corrigée, afin de pouvoir observer les tracés montrant des sur ou sous-estimations,
- indices dérivés ∆PP, dP/dt max,
- situation de la liaison hydraulique par rapport à ses limites de reproduction du signal dans une tolérance donnée,

- évolution dans le temps des paramètres dynamiques de la liaison,
- journal des évènements (purges, obstructions, artéfacts divers ...)

Ledit système peut également permettre de réaliser des opérations de calibrage et de test du dispositif de surveillance.

Il peut également permettre, dans un mode spécial de réglage, accessible par mot de passe, de modifier certains paramètres du module de surveillance (notamment les seuils de tolérance) et de mettre à jour les versions du logiciel embarqué dans ledit module.

Le système de traitement 300 peut être implémenté, avec un logiciel adapté, sur un dispositif de type Assistant Personnel portable (PDA), ordinateur portable ou appareil spécifique.

Il est doté d'un écran de visualisation graphique et d'un organe de saisie (clavier, écran tactile, etc.) permettant à l'utilisateur d'interagir avec le logiciel.

On va maintenant décrire de manière plus détaillée les différentes méthodes de traitement du signal susceptibles d'être mises en oeuvre par le module de surveillance.

Détermination des paramètres dynamiques de la liaison hydraulique

Cette détermination met en oeuvre différentes méthodes présentant chacune des forces et parfois des faiblesses.

Pour cette raison, le dispositif de surveillance s'appuie sur les valeurs obtenues en établissant de préférence un indice de confiance pour chacune d'entre elles et établit un résultat en sortie de ce processus.

*Méthode percussionnelle utilisant les purges* (non utilisé par la présente invention)

L'analyse percussionnelle consiste à envoyer un signal échelon en entrée du système, et à observer comment celui-ci évolue en sortie.

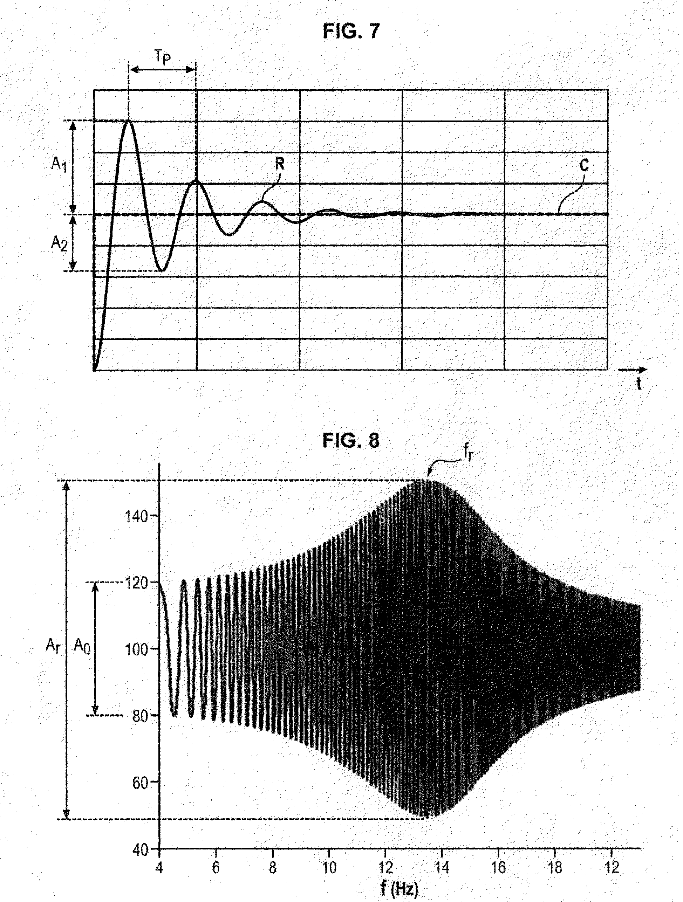

En référence à la figure 7, l'application d'un signal de consigne C sous forme d'un échelon donne lieu à un signal de réponse R.

Les caractéristiques de retour à la consigne (dépassements $A_1$, $A_2$ et fréquence d'oscillation fp qui est l'inverse de la période Tp) permettent de trouver les paramètres dynamiques du système, à savoir l'amortissement z et la fréquence propre $f_0$ grâce aux formules suivantes :

$$z = \frac{1}{\sqrt{1 + \left( \dfrac{\pi}{\ln(A_1 / A_2)} \right)^2}} \text{ et } f_0 = \frac{fp}{\sqrt{1 - z^2}}$$

Cette méthode met donc à profit les purges déclenchées par le personnel soignant et faisant partie du protocole, qui fournissent une source d'échelons à partir desquels la méthode peut être implémentée.

La réponse à l'échelon "purge" est intimement mêlée au signal hémodynamique du patient, il faut donc réaliser une opération d'extraction avant de pouvoir procéder à l'analyse indiquée ci-dessus.

Cette extraction est réalisée en dissociant, d'une part, le signal d'excitation de la purge et la réponse du système à cette purge, et d'autre part le signal de pression artérielle incident.

Elle permet ainsi une détermination précise et robuste des paramètres dynamiques $f_0$ et z de la liaison hydraulique.

Cependant, dans la mesure où les purges ne sont pas toujours effectuées de manière régulière (l'intervalle peut aller jusqu'à 8h voire plus), ni de de façon suffisamment énergique, et dans la mesure où une tubulure évoluant pendant plusieurs heures sans purge, différents phénomènes (ex. : microbulles ou autres) peuvent amener une dérive importante de $f_0$ et z, sans que la purge ne permette de les mettre en évidence, l'analyse percussionnelle basée sur les purges est de préférence utilisée en combinaison avec d'autres méthodes d'analyse.

Ainsi, par exemple, une première estimation de $f_0$ et z peut être tirée de l'extraction de la réponse de la liaison hydraulique à ces purges, puis affinée par l'une des méthodes décrites ci-après.

*Méthode percussionnelle utilisant le module actuateur*

Cette méthode, utilisé par la présente invention, est similaire à celle utilisant les purges, mais dans ce cas, la percussion est déclenchée par le module actuateur sous la forme d'une brève impulsion mécanique, sur une base temporelle régulière, ce qui permet de connaître $f_0$ et z entre deux purges du système.

Elle remédie ainsi à certains inconvénients de la méthode précédente basée sur les purges, notamment en offrant une périodicité de mise en oeuvre.

Comme la méthode précédente, elle nécessite également une extraction.

Cependant, du fait que le déclenchement de l'impulsion est piloté par le module de surveillance, l'action peut se situer dans une période moins accidentée du signal, par exemple en diastole.

Cette méthode est particulièrement avantageuse pour calculer la fréquence propre $f_0$.

*Méthode harmonique utilisant le module actuateur*

Cette méthode est particulièrement précise et reproductible et est donc préférentiellement mise en oeuvre, éventuellement combinée à l'une des méthodes précédentes.

L'analyse harmonique consiste à envoyer un train de signaux sinusoïdaux de fréquence variable en entrée de la liaison hydraulique.

Comme illustré à la figure 8, on obtient en sortie une courbe caractérisée par un maximum d'amplitude $A_r$ à la fréquence de résonance fr.

A partir de ces caractéristiques, on peut retrouver les paramètres dynamiques de la liaison hydraulique, grâce aux formules :

$$z = \sqrt{\frac{1 - \sqrt{1 - (A_0 / A_r)^2}}{2}} \text{ et } f_0 = \frac{fr}{\sqrt{1 - 2z^2}}.$$

Cette méthode nécessite la génération d'un train d'ondes sinusoïdales de fréquence variable présentant une perturbation plus importante pour le système de mesure.

Par conséquent, ladite méthode peut nécessiter de segmenter l'action mécanique en plusieurs salves réparties sur des diastoles successives.

Afin de faciliter l'analyse, cette méthode pourra avantageusement être synchronisée par le correcteur pour intervenir dans une phase peu perturbée du signal, telle qu'une diastole.

*Analyse de l'évolution du spectre du signal mesuré*

Le principe de cette méthode repose sur le fait que l'apparition de résonances au niveau de la liaison hydraulique impacte le contenu spectral du signal mesuré.

Par un traitement approprié, le système peut détecter une résonance et en évaluer les paramètres ($f_0$ et z) [11].

Les résultats issus de ce processus permettent de conforter les mesures issues des autres méthodes et de détecter l'évolution des caractéristiques dynamiques de la liaison hydraulique.

Détermination du contenu fréquentiel du signal incident

Cette détermination est effectuée sur la base d'une analyse FFT du signal incident pour établir d'une part la valeur de la fréquence fondamentale ff et d'autre part la valeur de la composante maximale à transmettre (fh).

fh est établie en évaluant l'étendue des harmoniques d'amplitude significative (apportant une contribution à l'analyse).

A cet égard, la majorité des auteurs s'accorde sur 5 à 10 harmoniques.

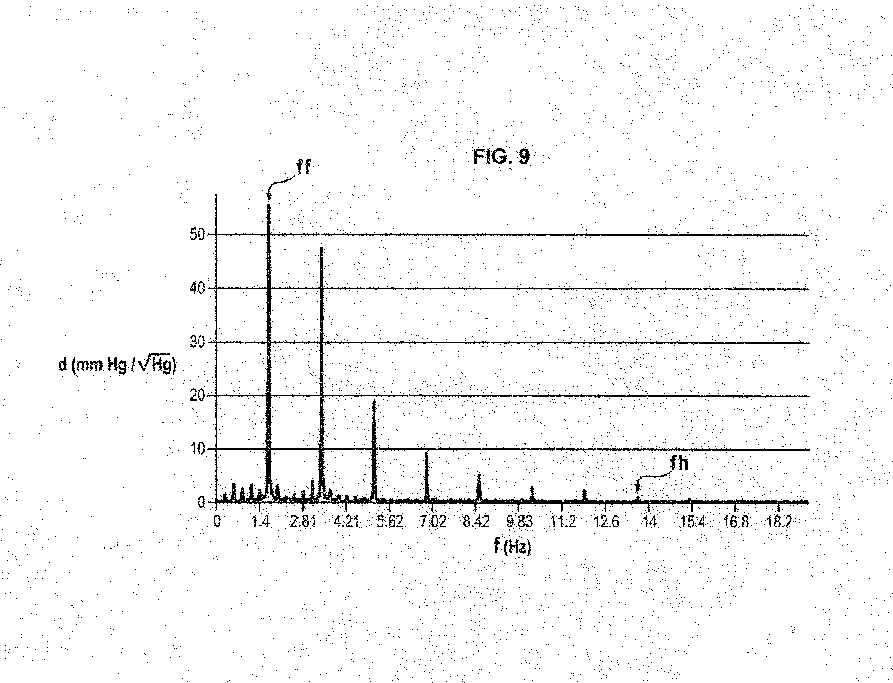

A titre purement indicatif, la figure 9 illustre une courbe de la densité spectrale du signal de pression en fonction de la fréquence.

Estimation de l'aptitude de la liaison hydraulique à reproduire fidèlement le signal incident

Cette estimation s'appuie sur la détermination préalable de f0, z et, le cas échéant, de fh.

La connaissance de $f_0$ et z suffit à caractériser la liaison hydraulique.

Pour évaluer correctement la capacité de ladite liaison à reproduire correctement un signal incident donné, plusieurs méthodes sont possibles.

Une première méthode comprend l'application, de façon logicielle, d'une correction du signal incident brut distordu suivie d'une étude comparative des deux signaux (brut et corrigé).

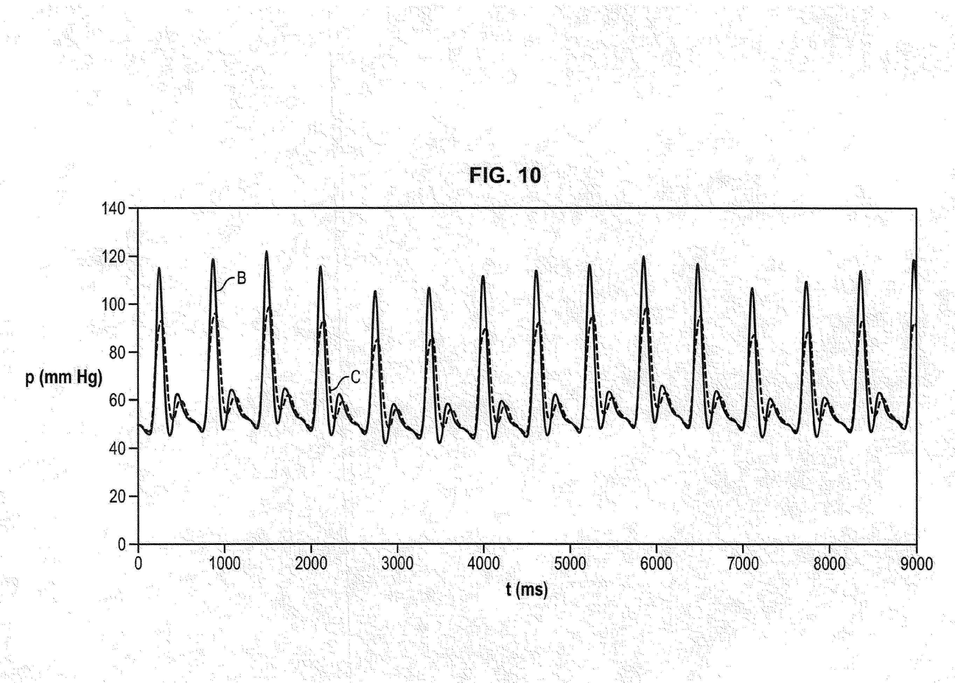

La figure 10 représente une portion de signal recueillie immédiatement après une purge, faisant apparaître le signal brut B et le signal corrigé C, ce qui a permis de déterminer par analyse percussionnelle les caractéristiques de la liaison hydraulique reliant le cathéter au capteur et ainsi de reconstruire le signal exempt de déformations.

Une deuxième méthode a été présentée par plusieurs auteurs [12].

Ladite méthode peut être illustrée par des gabarits ou abaques préétablis faisant apparaître, dans un graphique réalisé dans le plan ($f_0$, z), des zones où une composante de fréquence maximum fh pourra être ou non reproduite avec une distorsion inférieure à 5% par exemple.

Le positionnement sur l'abaque des paramètres de la liaison hydraulique ($f_0$, z) et de la composante de fréquence maximum du signal incident (fh), permet de déterminer si le dispositif est apte ou non à traiter le signal du patient.

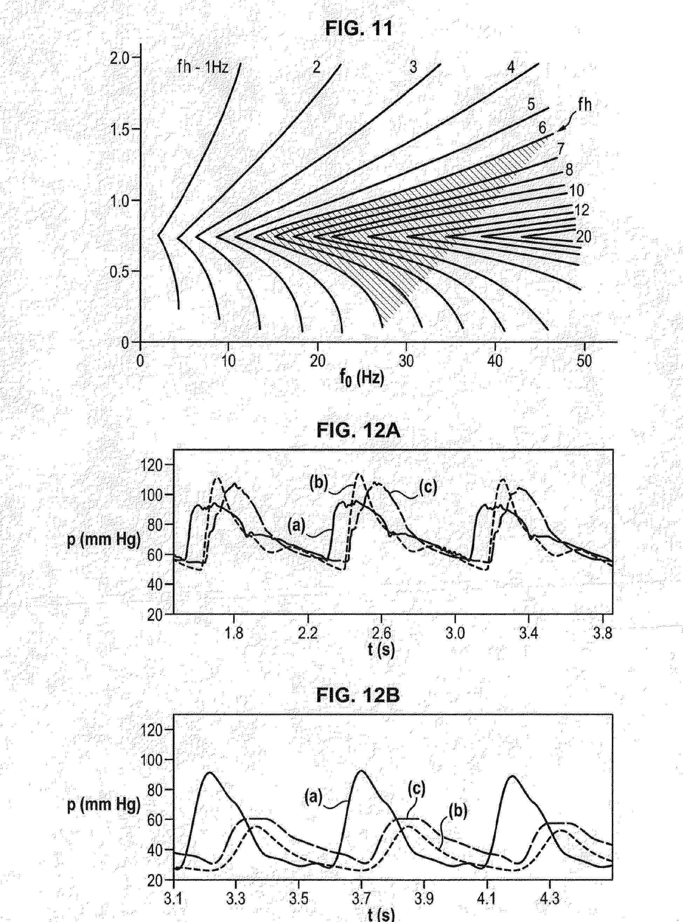

La figure 11 présente un exemple de mise en oeuvre de ladite méthode.

Pour une fréquence donnée fh maximum à transmettre (fixée ici, à titre d'exemple, à 6 Hz), l'aire comprise à l'intérieur de la courbe correspondant à cette fréquence représente, dans le plan ($f_0$, z), l'ensemble des valeurs compatibles avec la tolérance de $\pm$ 5% (hachurée sur le schéma).

Les deux méthodes décrites ci-dessus reposent sur des processus différents, mais doivent aboutir à des résultats cohérents.

Dans ce cas, l'estimation de la qualité de la liaison hydraulique est possible, tandis que dans le cas contraire, il est nécessaire de refaire une caractérisation de la liaison hydraulique.

Surveillance de la liaison hydraulique

Une des fonctions du dispositif de surveillance consiste à surveiller en permanence l'ensemble du système de mesure de la pression artérielle, dans le but de détecter les situations pouvant compromettre la fiabilité des indications affichées sur le moniteur patient

Lesdites situations sont les suivantes.

D'une part, des artéfacts de différentes catégories peuvent être détectés.

Il peut s'agir d'artéfacts de mouvement dus aux changements de position du patient, notamment les parties du corps proches du système de mesure (ex. : poignet en cas de cathétérisme de l'artère radiale) ; d'artéfacts dus à des interactions avec l'extérieur, par exemple lorsque le personnel soignant percute involontairement la tubulure, ce qui a pour traduction un à-coup au niveau du tracé de pression.

Ces artéfacts sont des phénomènes affectant les mesures de façon temporaire.

Le dispositif de surveillance les détecte et informe le personnel soignant, par exemple par un clignotement bref de couleur rouge du voyant d'état.

Une autre fonction du dispositif de surveillance est de détecter des atténuations anormales du signal incident.

Le phénomène d'atténuation du signal, déjà décrit dans la littérature [13], se traduit par un tracé affaibli souvent de façon notable et quelquefois qualifié abusivement d'amortissement, phénomène différent dont il est à distinguer.

En effet, un sur-amortissement de la liaison hydraulique peut intervenir lorsque le coefficient d'amortissement z atteint une valeur trop élevée, suite par exemple à l'accumulation de microbulles dans la tubulure.

Ce phénomène est caractérisé par le dispositif de surveillance et peut, dans certains cas, faire l'objet d'une correction automatique et/ou d'une alerte du personnel soignant sur la nécessité d'une intervention.

En revanche, dans le cas du phénomène d'atténuation, l'origine du défaut ne se situe pas au niveau de la liaison hydraulique elle-même, mais au niveau du cathéter ou de l'artère du patient où un obstacle temporaire (exemple : caillot, ...) peut apparaître, voire précéder le cathétérisme (par exemple, une plaque d'athérome dans les artères du patient).

Il en résulte une obstruction temporaire ou durable, entraînant une atténuation importante du signal rendant la mesure inexploitable.

Ce type de défaut ne peut pas être corrigé automatiquement par le dispositif de surveillance.

Cependant, la fonction de surveillance de ce dispositif détecte cette situation et informe le personnel soignant par un allumage du voyant d'état, par exemple en rouge fixe, superposé à un éclair lumineux périodique plus intense, destiné à attirer l'attention sur l'urgence d'une intervention.

Cette intervention se traduit le plus souvent par une purge qui peut faire disparaître le défaut.

Dans ce cas, le dispositif détecte le retour à la normale avec un voyant d'état allumé en vert ou orange.

Dans le cas où le défaut n'a pas disparu ou réapparaît au bout d'un certain temps, le dispositif sollicite de nouveau une intervention qui peut alors être plus lourde, comme le remplacement d'un cathéter.

L'avantage de la détection procuré par le dispositif de surveillance est de ne pas attendre que le tracé soit complètement atténué avant de donner l'alerte.

D'autre part, le dispositif de surveillance peut consigner les incidents intervenus sur la mesure de pression artérielle.

Ainsi, chaque incident ayant affecté temporairement ou durablement la fiabilité des mesures pendant la durée de vie du dispositif de mesure est consigné dans un journal mémorisé dans le module de surveillance.

La consultation de ce journal peut être faite au moyen du système de traitement mentionné plus haut.

La réinitialisation du journal (à l'ablation du cathéter, par exemple), peut être faite par une commande du système de traitement ou, par exemple, un appui long sur le bouton de mode.

En l'absence de réinitialisation, les incidents continuent à s'inscrire jusqu'à concurrence du remplissage de la mémoire.

Cette limite atteinte, les événements les plus anciens sont automatiquement purgés pour laisser la place aux plus récents.

Détection d'un découplage entre la pression artérielle centrale et la pression artérielle périphérique

Le dispositif décrit plus haut peut avantageusement être complété d'un module de détection d'un éventuel découplage entre la pression artérielle périphérique (mesurée par cathétérisme de l'artère radiale) et la pression artérielle centrale ou aortique.

En effet, certains contextes cliniques particuliers (choc septique, voire sortie de circulation extracorporelle) sont caractérisés par une modification physiopathologique majeure des caractéristiques de l'arbre artériel périphérique.

Cette modification est susceptible de rendre inutilisable la mesure de la pression artérielle par cathétérisme de l'artère radiale qui ne reflète alors plus la pression artérielle centrale aortique.

Or, celle-ci commande des organes aussi importants que le coeur, le cerveau, etc.

Le monitorage du niveau moyen de la pression artérielle centrale est donc un élément important du diagnostic, de la surveillance et du pilotage médicamenteux des patients, notamment de ceux en état de choc septique [14].

Par ailleurs, la forme et l'amplitude de la pression artérielle périphérique sont utilisées comme signal d'entrée des appareils de mesure de débit cardiaque en continu, permet l'évaluation de la volémie fonctionnelle sous ventilation mécanique ainsi que celle d'autres paramètres (contractilité cardiaque, etc.) [3].

Un module de détection du découplage entre pressions artérielles centrale et périphérique présente donc un intérêt particulier dans le monitorage de ces situations cliniques.

Les figures 12A et 12B illustrent la variation de la pression artérielle aortique (courbe (a)), radiale (courbe (b)) et fémorale (courbe (c)), respectivement dans une situation normale et dans une situation de choc septique [15].

A l'état normal, la pression artérielle radiale présente un pic systolique d'amplitude supérieure à celle de la pression aortique, notamment en raison des réflexions dues aux caractéristiques de l'arbre artériel périphérique.

En état de choc septique, ce gradient de pression s'inverse.

Il est à noter que cette diminution du pic systolique entraîne à son tour une diminution modérée du niveau moyen de la pression artérielle périphérique radiale par rapport à celui de la pression aortique, cette diminution devenant cliniquement significative dans certains cas [14].

Cette modification peut s'expliquer par le mécanisme suivant.

Un patient en choc septique conserve une compliance artérielle centrale proche de la normale.

En revanche, il voit sa compliance artérielle périphérique augmenter en raison de la richesse des parois des artères périphériques en fibres musculaires lisses, sensibles aux médiateurs libérés dans le choc septique et aux modifications du tonus sympathique.

Ces effets sont beaucoup moins importants au niveau des artères centrales élastiques, peu riches en fibres musculaires lisses.

La détection du découplage peut être réalisée par deux méthodes différentes, qui peuvent être complémentaires, et qui reposent sur des principes différents d'Interprétation des signaux de pression.

Outre le capteur 3 relié au cathéter radial par la liaison hydraulique 2, ces méthodes mettent en oeuvre une tonométrie de l'artère humérale/brachiale (au niveau du coude), voire une tonométrie de l'artère carotidienne.

*Analyse fréquentielle*

Une première méthode repose sur une analyse fréquentielle.

L'augmentation de la compliance artérielle périphérique entraîne une répercussion sur la réponse en fréquence de l'arbre artériel périphérique assimilable à celle d'un un filtre passe-bas (qui atténue les composantes de haute fréquence).

Pour un même signal d'entrée (pression aortique), on observe un signal de sortie (pression artérielle radiale) différent, selon que l'on se trouve en situation normale ou en cas de choc septique.

Dans le cas du choc septique, les harmoniques de rang supérieur se trouveront réduites relativement aux harmoniques de rang inférieur.

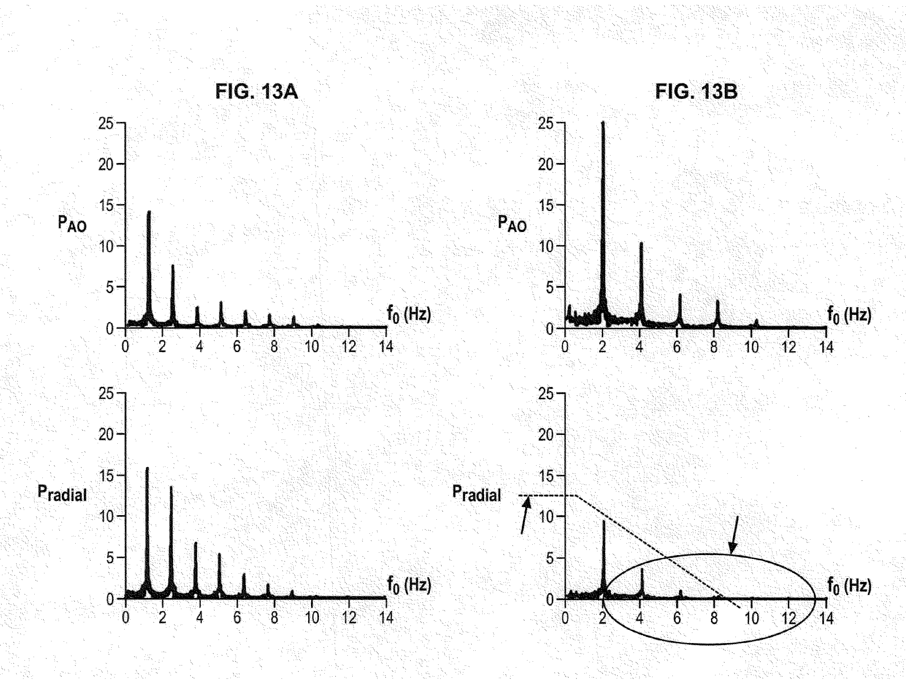

Les figures 13A et 13B (issues de [15]) illustrent respectivement les pressions aortique PAo et radiale Pradial chez un porc normal et chez un porc en situation de choc septique.

Dans le cas normal, on observe une conservation du contenu spectral entre la pression aortique et la pression radiale.

En revanche, dans le cas d'un choc septique, on observe une forte atténuation des harmoniques de rang supérieur sur la pression artérielle radiale (zone entourée).

La ligne en pointillés illustre l'effet de type « passe-bas » mentionné plus haut.

Une surveillance en continu du contenu spectral du signal de pression artérielle radiale permet d'observer, le cas échéant, une évolution de ce spectre vers un affaiblissement des harmoniques de rang élevé.

Il n'est pas possible d'en déduire que cela vient obligatoirement d'une augmentation de la compliance artérielle périphérique.

En effet, la cause peut en être également un changement du contenu spectral du signal d'entrée (pression aortique PAo).

Pour choisir entre ces deux interprétations, il faut :

- connaître le contenu spectral du signal d'entrée (pression aortique ou pression artérielle humérale),
- mesurer le ratio RHAo = harmoniques supérieures / harmoniques inférieures du signal d'entrée.

Comme la pression aortique n'est pas accessible en pratique clinique courante, on peut approcher cette mesure par la pression artérielle humérale voire par la pression artérielle carotidienne si le lieu de mesure par tonométrie de cette dernière est accessible.

Comme il s'agit d'un rapport d'amplitudes, une mesure par tonométrie de la pression artérielle humérale ou carotidienne est suffisante.

- comparer le ratio RHao avec le même ratio RHrad mesuré en artère radiale.

Un écart important (supérieur à un seuil prédéterminé) fournit un indice du découplage entre pression artérielle centrale et périphérique.

*Mesure de la vitesse de progression de l'onde de pouls*

Une seconde méthode repose sur la mesure de la vitesse de progression de l'onde de pouls.

Certaines pathologies entraînent des modifications du tonus des parois des artères périphériques, dont la richesse en fibres musculaires lisses par rapport aux artères centrales a déjà été soulignée.

Dans les situations cliniques de choc septique ou de sortie de circulation extracorporelle, il en résulte une augmentation de la compliance artérielle périphérique [15].

Cette augmentation de la compliance des parois artérielles périphériques peut être mesurée par la diminution de la vitesse de progression de l'onde de pouls (PWV, acronyme du terme anglo-saxon « Pulse Wave Velocity »).

Les paramètres qui influent sur cette vitesse se répartissent entre les changements de l'élasticité de la paroi artérielle (module de Young incrémental (Einc), de l'épaisseur de cette paroi (h), et du diamètre de l'artère radiale (RAD), en utilisant l'équation de Moens-Korteweg où ρ représente la densité du sang :

$$PWV = \sqrt{\frac{Einc.\,h}{\rho.\,RAD}}$$

A défaut d'une illustration dans la littérature de la diminution de la vitesse de propagation de l'onde de pouls dans le domaine du choc septique, il est possible d'en trouver dans les études pharmacologiques et dans les sorties de circulation extracorporelle au cours des interventions de chirurgie cardiaque.

Ainsi, Fok et al. observent un ralentissement de la progression de l'onde de pouls lorsqu'ils injectent des substances vasodilatatrices telles que les donneurs de NO (« Nitric Oxide Donors ») dans l'artère humérale en amont de l'artère radiale [16].

Or le choc septique donne lieu à la libération de ce type de médiateurs.

Ainsi, Kanasawa et al montrent que, chez certains de leurs patients en sortie de circulation extracorporelle, l'amplitude et le niveau moyen de la pression artérielle diminuent progressivement à mesure que le lieu de mesure de la pression artérielle s'éloigne de l'aorte vers l'artère radiale [17].

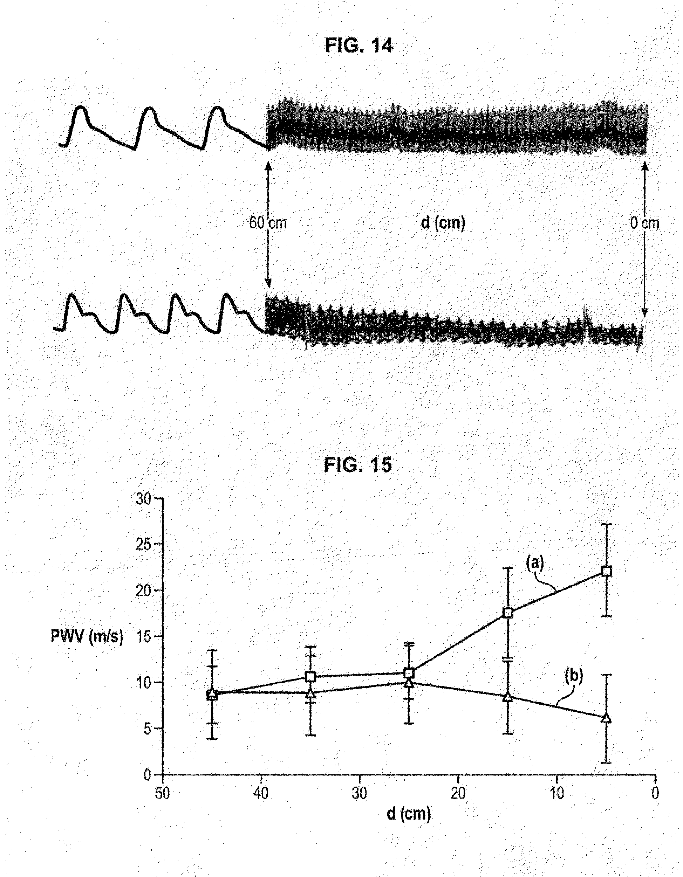

La figure 14 illustre respectivement la distribution de la pression artérielle de l'aorte vers l'artère radiale chez l'un de ces patients avant (courbe du haut) et après (courbe du bas) la mise en oeuvre d'une circulation extracorporelle, en fonction de la distance d par rapport à l'artère radiale.

Ce groupe de patients présente donc un gradient de pression entre la pression artérielle centrale et la pression artérielle périphérique.

Dans ce groupe de patients présentant un gradient entre la pression artérielle centrale et la pression artérielle périphérique, la vitesse de propagation de l'onde de pouls diminue, comme le montre la figure 15 [18].

La figure 15 illustre la vitesse moyenne de propagation de l'onde de pouls en fonction de la distance par rapport à l'artère radiale, avant (courbe (a)) et après (courbe (b)) la mise en oeuvre d'une circulation extracorporelle chez les patients mentionnés ci-dessus.

Le principe de la seconde méthode de détection du découplage consiste à mesurer l'intervalle de temps séparant les pics systoliques ou le pied de ces pics systoliques mesurés par tonométrie de l'artère humérale/brachiale, d'une part, et par cathétérisme de l'artère radiale d'autre part.

Cet intervalle de temps permet au dispositif de surveillance selon l'invention, augmenté de l'option de détection du découplage entre pression artérielle centrale et périphérique, d'estimer la vitesse actuelle de propagation de l'onde de pouls et de la comparer à des valeurs normales de référence connues.

Elle nécessite toutefois la saisie par l'opérateur de la distance humérale/radiale au moyen d'un réglage incorporé au dispositif de tonométrie ou au dispositif de surveillance lui-même.

Une variante de cette méthode utilise deux mesures tonométriques : une mesure humérale/brachiale et une mesure carotidienne.

L'impact d'une situation de découplage sur le temps de propagation carotide/humérale étant généralement moindre, alors qu'il est important sur le temps de propagation humérale/radiale, l'exploitation de ce déséquilibre fournit un indice permettant la détection du découplage.

Une autre variante de cette méthode peut être utilisée en cas d'inaccessibilité de l'emplacement où s'applique la tonométrie carotidienne chez un patient hospitalisé en soins intensifs (pansement, accès veineux jugulaire interne, etc.).

Une fonction de transfert permet alors de calculer la pression aortique à partir de la tonométrie de l'artère humérale [3].

L'une et/ou l'autre des méthodes de détection du découplage sont mises en oeuvre au moyen d'un boîtier tonométrique qui peut être indépendant du module de surveillance et de correction.

Ce boîtier comprend typiquement un capteur de mesure de pression artérielle par tonométrie, une alimentation par batterie rechargeable, un dispositif d'affichage et des boutons étanches permettant à l'opérateur d'interagir avec le dispositif.

Ce boîtier est en liaison sans fil ou filaire avec le module de surveillance et de correction.

Le fonctionnement de ce dispositif complémentaire est le suivant.

Le dispositif de surveillance et de correction muni de cette option surveille en permanence la pression artérielle radiale à travers la liaison hydraulique (fonctions de base décrites plus haut).

Lorsque l'option de détection du découplage entre pressions centrale et périphérique est en service, le dispositif effectue, en permanence également, une fonction supplémentaire qui consiste à surveiller le rapport RHrad (harmoniques supérieures/harmoniques inférieures) du signal mesuré.

Si ce rapport s'effondre brutalement, le dispositif génère un avertissement spécifique.

Le praticien muni du boîtier tonométrique effectue alors une mesure de tonométrie en artère humérale et carotidienne accompagnée d'une saisie de la (des) distances humérale/radiale (et carotidienne/radiale).

Cette saisie s'effectue au moyen des touches et du dispositif d'affichage incorporés au boîtier tonométrique.

En cas d'impossibilité d'effectuer la tonométrie carotidienne (pansement, accès veineux jugulaire interne, etc.), le tracé de pression aortique est évalué par une fonction de transfert à partir du tracé de la pression humérale [3].

Le dispositif met alors en oeuvre les méthodes d'analyse fréquentielle et d'analyse de la vitesse de progression de l'onde de pouls décrites précédemment.

Si les deux méthodes ne détectent aucune situation anormale, l'avertissement est annulé.

Si les deux méthodes fournissent des résultats divergents, le praticien est invité à reprendre la (les) mesures tonométriques.

Si les deux méthodes conduisent à une détection positive indiquant une situation où la mesure de la pression artérielle par cathétérisme de l'artère radiale n'est plus le reflet de la pression centrale, l'avertissement est transformé en alarme. Ce dispositif de fusion des données est original.

Enfin, il va de soi que les exemples que l'on vient de donner ne sont que des illustrations particulières en aucun cas limitatives quant aux domaines d'application de l'invention.

Par ailleurs, il est bien entendu que la présente invention ne porte en aucun cas sur la mise en place du cathéter dans l'artère du patient, mais uniquement sur un procédé et un dispositif permettant, une fois ledit cathéter introduit dans l'artère du patient et relié au capteur de pression par la liaison hydraulique, de surveiller et, le cas échéant, de corriger, le signal de mesure de pression fourni par le capteur.

## REFERENCES

[1] M. Cannesson, Arterial Pressure Variation and Goal-Directed Fluid Therapy. J Cardiothorac Vase Anesth. 24 (3), 2010, pp. 487-497

[2] J. Mayer, J. Boldt, R. Poland, A. Peterson et G. Manecke, Continuous Arterial Pressure Waveform-Based Cardiac Output Using the FloTrac/Vigileo: A Review and Meta-analysis. J Cardiothorac Vasc Anesth. 23 (3), 2009, pp. 401-406

[3] R. Thiele, ME. Durieux, Arterial waveform analysis for the anesthesiologist: past, présent, and future concepts. Anesth Analg. 113 (4), 2011, pp. 766-776

[4] M. Gardner, Direct Blood Pressure Measurement - Dynamic Response Requirements, Anesthesiology, 54, 1981, pp. 227-236

[5] C. Promonet, D. Anglade, A. Menaouar, S. Bayat, M. Durand, A. Eberhard et F. Grimbert, Time-dependent Pressure Distortion in a Catheter-Transducer System, Anesthesiology, 92 (1), 2000, pp. 208-218.

[6] M. Allan, W. Gray, J. Asbuη, Measurement of Arterial Pressure Using Catheter-Transducer Systems, BrJ Anaesth, 60, 1988, pp. 413-418.

[7] B. Kleinman, Understanding natural frequency and damping and how they relate to the measurement of blood pressure, J Clin Monit, 5 (2), 1989, pp. 137-147.

[8] E. Billiet et F. Colardyn, Pressure measurement evaluation and accuracy validation: the Gabarith test, Int Care Med. 24 (12), 1998, pp. 1323-1326.

[9] A.W.Paulsen, Implications for clinical monitoring of intra-arterial blood pressure based on the frequency content of worst-case pressure waveforms, Medical Instrumentation & Technology, May-June 1993, pp. 217-234.

[10] Brevet DE 39 27 990, 28 fév. 1991.

[11] R. Brower, W. Spaans, P. Rewiersma et G. Meester, A fully automatic device for compensating for artifacts in conventional catheter-manometer pressure recordings, Biomed Eng,. 10, 1975, pp. 305-310.

[12] Y. Kinefuchi, T. Suzuki, M. Takiguchi, Y. Yamasaki et M. Yamamoto, Evaluation of dynamic response of catheter-manometer systems for pulmonary arterial pressure, J Appl Physiol, 77 (4), 1994, pp. 2023-2028.

[13] A. Ercole, Atténuation in invasive blood pressure measurement system, Br J Anaesth. 96 (5), 2006, pp. 560-562.

[14] Dellinger RP, Levy MM, Carlet JM, Bion J, Parker MM, Jaeschke R, Reinhart K, Angus DC, Brun-Buisson C, Beale R, Calandra T, Dhainaut JF, Gerlach H, Harvey M, Marini JJ, Marshall J, Ranieri M, Ramsay G, Sevransky J, Thompson BT, Townsend S, Vender JS, Zimmerman JL, Vincent JL; International Surviving Sepsis Campaign Guidelines Committee; American Association of Critical-Care Nurses; American College of Chest Physicians; American College of Emergency Physicians; Canadian Critical Care Society; European Society of Clinical Microbiology and Infectious, Diseases; European Society of Intensive Care Medicine; European Respiratory, Society; International Sepsis Forum; Japanese Association for Acute Medicine;, Japanese Society of Intensive Care Medicine; Society of Critical Care Medicine;, Society of Hospital Medicine; Surgical Infection Society; World Fédération of Societies of Intensive and Critical Care Medicine. Surviving Sepsis Campaign: international guidelines for management of severe sepsis and septic shock: 2008. Crit Care Med. 2008 Jan;36(1):296-327. Erratum in: Crit Care Med. 2008 Apr;36(4):1394-6

[15] F. Hatib et al, Peripheral vascular decoupling in porcine endotoxic shock, J. Appl. Physiol. 111 : 853-860

[16] H. Fok et al, Régulation of Vascular Tone and Pulse Wave Velocity in Human Muscular Conduit Arteries - Sélective Effects of Nitric Oxide Donors to Dilate Muscular Arteries Relative to Résistance Vessels, Hypertension, Nov. 2012, pp. 1220-1225

[17] M. Kanazawa, Relationship between Aortic-to-radial Arterial Pressure Gradient after Cardiopulmonary Bypass and Changes in Arterial Elasticity, Anestesiology, V. 99, No.1, Jul. 2003

[18] M. Shimada et al, Estimation of Aortic-to-radial Artery Distribution of Arterial Wall Elasticity, Tokai J. Exp. Clin. Med., Vol. 33, No. 1, pp 1-5, 2008

**Revendications**

1. Procédé de surveillance en continu de la mesure de la pression artérielle effectuée par un capteur de pression (3) relié, par l'intermédiaire d'une liaison hydraulique (2), à un cathéter (1) préalablement introduit dans l'artère d'un patient, ladite liaison hydraulique (2) transmettant au capteur (3) un signal de pression artérielle du patient, dit « signal incident » appliqué à l'extrémité intra-artérielle du cathéter (1) et le capteur (3) transformant le signal transmis par la liaison hydraulique (2) en un signal électrique, dit « signal mesuré », ledit procédé comprenant les étapes suivants, réalisés par au moins un processeur (101, 102), de :

- détermination, à partir du signal mesuré, des paramètres dynamiques de ladite liaison hydraulique (2), lesdits paramètres comprenant la fréquence propre ($f_0$) et le coefficient d'amortissement (z) de la liaison hydraulique (2),
- analyse du contenu fréquentiel du signal incident, de manière à déterminer la composante de fréquence maximale (fh) dudit signal,
- détection, à partir desdits paramètres dynamiques de la liaison hydraulique (2) et de la fréquence maximale à transmettre, d'une situation parmi les situations suivantes :

(a) l'aptitude de la liaison hydraulique (2) à transmettre le signal incident au capteur (3) avec une distorsion inférieure à un seuil,
(b) l'aptitude de la liaison hydraulique (2) à transmettre le signal incident au capteur (3) avec une distorsion supérieure audit seuil et la possibilité de corriger le signal mesuré,
(c) l'inaptitude de la liaison hydraulique (2) à transmettre le signal incident au capteur (3) avec une distorsion inférieure audit seuil et l'impossibilité de corriger le signal mesuré,

- dans le cas où la situation (c) est détectée, émission d'un signal d'alerte par l'intermédiaire d'une interface homme-machine,

ledit procédé comprenant en outre l'application périodique, par un module actuateur (200), d'une action mécanique sur la paroi externe de la tubulure qui assure la liaison hydraulique (2) et l'analyse, par le processeur, du signal mesuré en réponse à ladite action mécanique pour déterminer les paramètres dynamiques ($f_0$, z) de ladite liaison hydraulique, le processeur mettant en oeuvre, pour la détection de l'une des situations (a), (b) ou (c) :

(i) une comparaison du signal mesuré et d'un signal mesuré corrigé en fonction des paramètres dynamiques ($f_0$, z) de la liaison hydraulique (2),
(ii) le positionnement, sur un abaque fréquence propre-amortissement, du point représentatif des paramètres dynamiques (f0, z) de la liaison hydraulique par rapport à une aire de tolérance déterminée par la composante de fréquence maximale du signal utilisé (fh), et
(iii) une vérification de la cohérence des résultats des étapes (i) et (ii).

2. Procédé selon la revendication 1, **caractérisé en ce que** ladite action mécanique comprend une percussion de la paroi externe de la tubulure qui assure la liaison hydraulique et/ou l'application d'un train de sollicitations sinusoïdales de fréquence variable à la paroi externe de la tubulure qui assure la liaison hydraulique.

3. Procédé selon l'une des revendications 1 à 2, **caractérisé en ce que** ladite action mécanique est synchronisée avec une diastole ou segmentée de manière synchronisée avec plusieurs diastoles successives.

4. Procédé selon l'une des revendications 1 à 3, **caractérisé en ce que** dans le cas où la situation (b) est détectée, ledit procédé comprend la correction du signal mesuré en fonction des paramètres dynamiques ($f_0$, z) de la liaison hydraulique (2).

5. Procédé selon l'une des revendications 1 à 4, **caractérisé en ce qu'**il comprend l'enregistrement du signal mesuré ou, dans la situation (b), d'un signal corrigé et/ou la transmission dudit signal à un système de traitement.

6. Procédé selon l'une des revendications 1 à 5, **caractérisé en ce que** si ladite analyse du contenu fréquentiel du signal incident détecte un glissement spectral et/ou une variation du poids relatif des composantes de fréquences supérieures, on met en oeuvre ladite étape de détermination des paramètres dynamiques ($f_0$, z) de la liaison hydraulique (2) de manière à réactualiser lesdits paramètres.

7. Procédé selon l'une des revendications 1 à 6, **caractérisé en ce qu'**il comprend en outre la détection d'un découplage entre la pression artérielle centrale et la pression artérielle radiale du patient, ladite détection comprenant la mise en oeuvre d'une mesure tonométrique de l'artère humérale/brachiale et/ou carotidienne, la surveillance de la vitesse de propagation de l'onde de pouls du patient déterminée à partir de ladite mesure tonométrique et du signal mesuré, et la comparaison de ladite vitesse avec une valeur de référence.

8. Procédé selon la revendication 7, **caractérisé en ce qu'**en cas d'impossibilité d'effectuer une mesure tonométrique carotidienne, on évalue la pression aortique par une fonction de transfert à partir de la mesure de la pression humérale.

9. Procédé selon la revendication 8 en combinaison avec la revendication 10, **caractérisé en ce que** l'on met en oeuvre chacune des dites étapes de détection d'un découplage et **en ce que**, si chacune des deux détections met en évidence un découplage de la pression artérielle centrale et de la pression artérielle radiale du patient, une alarme est émise.

10. Dispositif (100, 200, 300) de surveillance en continu de la mesure de la pression artérielle effectuée par un capteur de pression (3) relié, par l'intermédiaire d'une liaison hydraulique (2), à un cathéter (1) préalablement introduit dans l'artère d'un patient, ladite liaison hydraulique (2) transmettant au capteur (3) un signal de pression artérielle du patient, dit « signal incident » appliqué à l'extrémité intra-artérielle du cathéter (1), et le capteur (3) transformant le signal transmis par la liaison hydraulique (2) en un signal électrique, dit « signal mesuré », ledit dispositif comprenant:

   - un module de surveillance et de correction (100) comprenant au moins un processeur (101, 102), ledit module étant adapté pour mettre en oeuvre les étapes consistant à :

     - déterminer, à partir du signal mesuré, les paramètres dynamiques de ladite liaison hydraulique (2), lesdits paramètres comprenant la fréquence propre ($f_0$) et le coefficient d'amortissement (z) de la liaison hydraulique (2),
     - analyser le contenu fréquentiel du signal incident, de manière à déterminer la composante de fréquence maximale à transmettre (fh) dudit signal,
     - détecter, à partir desdits paramètres dynamiques de la liaison hydraulique (2) et de la fréquence maximale à transmettre, une situation parmi les situations suivantes :

       (a) l'aptitude de la liaison hydraulique (2) à transmettre le signal incident au capteur (3) avec une distorsion inférieure à un seuil,
       (b) l'aptitude de la liaison hydraulique (2) à transmettre le signal incident au capteur (3) avec une distorsion supérieure audit seuil et l'aptitude dudit module à corriger le signal mesuré,
       (c) l'inaptitude de la liaison hydraulique (2) à transmettre le signal incident au capteur (3) avec une distorsion inférieure audit seuil et l'inaptitude dudit module à corriger le signal mesuré,

   - une interface homme machine couplée au processeur pour, dans le cas où la situation (c) est détectée, émettre un signal d'alerte,
   - un module actuateur (200), adapté de sorte à appliquer de manière périodique une action mécanique sur la paroi externe de la tubulure qui assure la liaison hydraulique (2), le module de surveillance et de correction étant configuré pour analyser le signal mesuré en réponse à ladite action mécanique et pour déterminer les paramètres dynamiques ($f_0$, z) de ladite liaison hydraulique à partir de ladite analyse,

le module de surveillance et de correction étant configuré pour mettre en oeuvre, pour la détection de l'une des situations (a), (b) ou (c) :

   (i) une comparaison du signal mesuré et d'un signal mesuré corrigé en fonction des paramètres dynamiques ($f_0$, z) de la liaison hydraulique (2),
   (ii) le positionnement, sur un abaque fréquence propre-amortissement, du point représentatif des paramètres dynamiques (f0, z) de la liaison hydraulique par rapport à une aire de tolérance déterminée par la composante de fréquence maximale du signal utilisé (fh), et
   (iii) une vérification de la cohérence des résultats des étapes (i) et (ii).

**11.** Dispositif selon la revendication 10, **caractérisé en ce que** le module de surveillance et de correction (100) comprend :

> - un premier processeur (101), adapté pour déterminer les paramètres dynamiques ($f_0$, z) de la liaison hydraulique (2), analyser le contenu fréquentiel du signal incident, détecter l'une des situations (a), (b) ou (c) et, dans la situation (b), définir des paramètres de correction du signal mesuré en fonction des paramètres dynamiques ($f_0$, z) de la liaison hydraulique (2),
> - un second processeur (102), adapté pour, dans la situation (b), corriger en continu le signal mesuré à partir des paramètres de correction définis par le premier processeur (101).

**12.** Dispositif selon l'une des revendications 9 à 10, **caractérisé en ce que** le module actuateur (200) comprend un actionneur piézoélectrique ou un dispositif électromécanique.

**13.** Dispositif selon l'une des revendications 10 à 12, **caractérisé en ce qu'**il comprend une liaison filaire entre le module de surveillance et de correction (100) et le module actuateur (200), ladite liaison filaire étant adaptée pour fournir au module actuateur (200) un signal de synchronisation avec une diastole et/ou de l'énergie électrique en provenance du module de surveillance et de correction (100).

**14.** Dispositif selon l'une des revendications 10 à 12, **caractérisé en ce qu'**il comprend une liaison sans fil entre le module de surveillance et de correction (100) et le module actuateur (200), ladite liaison sans fil étant adaptée pour fournir au module actuateur (200) un signal de synchronisation avec une diastole émis par le module de surveillance et de correction (100).

**15.** Dispositif selon l'une des revendications 10 à 14, **caractérisé en ce que** le module actuateur (200) est adapté pour être rendu solidaire de la liaison hydraulique (2) à distance du module de surveillance et de correction (100).

**16.** Dispositif selon l'une des revendications 10 à 15, **caractérisé en ce qu'**il comprend un système de traitement (300) adapté pour communiquer avec le module de surveillance et de correction (100), ledit système de traitement (300) étant adapté pour traiter le signal mesuré, ou, dans la situation (b), un signal corrigé, transmis par le module de surveillance et de correction (100).

**17.** Dispositif selon l'une des revendications 10 à 16, **caractérisé en ce qu'**il comprend en outre un boîtier de mesure tonométrique d'une artère humérale et/ou d'une artère carotidienne.

## Patentansprüche

**1.** Verfahren zur kontinuierlichen Überwachung der Messung des arteriellen Drucks durch einen Drucksensor (3), der über eine hydraulische Verbindung (2) mit einem zuvor in die Arterie eines Patienten eingeführten Katheter (1) verbunden ist, wobei die hydraulische Verbindung (2) an den Sensor (3) ein Signal des arteriellen Drucks des Patienten, bezeichnet als "einfallendes Signal", überträgt, das am intraarteriellen Ende des Katheters (1) anliegt, und der Sensor (3) das durch die hydraulische Verbindung (2) übertragene Signal in ein elektrisches Signal, bezeichnet als "gemessenes Signal", umwandelt, wobei das Verfahren die folgenden Schritte umfasst, die von mindestens einem Prozessor (101, 102) durchgeführt werden:

> - Bestimmen, ausgehend von dem gemessenen Signal, der dynamischen Parameter der hydraulischen Verbindung (2), wobei die Parameter die Eigenfrequenz ($f_0$) und den Dämpfungskoeffizienten (z) der hydraulischen Verbindung (2) umfassen,
> - Analysieren des Frequenzinhalts des einfallenden Signals, um die maximale Frequenzkomponente (fh) des Signals zu bestimmen,
> - Ermitteln, ausgehend von den dynamischen Parametern der hydraulischen Verbindung (2) und von der zu übermittelnden maximalen Frequenz, einer Situation aus den folgenden Situationen:
>
> > (a) die Fähigkeit der hydraulischen Verbindung (2) zur Übertragung des einfallenden Signals an den Sensor (3) mit einer Verzerrung unter einem Grenzwert,
> > (b) die Fähigkeit der hydraulischen Verbindung (2) zur Übertragung des einfallenden Signals an den Sensor (3) mit einer Verzerrung über dem Grenzwert und der Möglichkeit, das gemessene Signal zu korrigieren,
> > (c) die Unfähigkeit der hydraulischen Verbindung (2) zur Übertragung des einfallenden Signals an den Sensor (3) mit einer Verzerrung unter dem Grenzwert und der Unmöglichkeit, das gemessene Signal zu korrigieren,
>
> - bei Ermittlung der Situation (c), Senden eines Warnsignals über eine Mensch-Maschine-Schnittstelle,
> wobei das Verfahren ferner die periodische Anwendung durch ein Aktuatormodul (200) einer mechanischen Aktion auf die Außenwand des Schlauchs umfasst, der die hydraulische Verbindung (2) gewährleistet, und die Analyse, durch den Prozessor, des gemessenen Signals als Antwort auf die mechanische Aktion, um die dynamischen Parameter ($f_0$, z) der hydraulischen Verbindung zu bestimmen,

wobei der Prozessor für die Ermittlung einer der Situationen (a), (b) oder (c) durchführt:

    (i) einen Vergleich des gemessenen Signals und eines korrigierten gemessenen Signals in Abhängigkeit von den dynamischen Parametern ($f_0$, z) der hydraulischen Verbindung (2),

    (ii) die Positionierung, auf einem Eigenfrequenz-Dämpfungs-Nomogramm, des repräsentativen Punkts der dynamischen Parameter ($f_0$, z) der hydraulischen Verbindung im Verhältnis zu einem von der maximalen Frequenzkomponente des verwendeten Signals (fh) bestimmten Toleranzbereich, und

    (iii) eine Überprüfung der Kohärenz der Ergebnisse der Schritte (i) und (ii).

**2.** Verfahren nach Anspruch 1, **dadurch gekennzeichnet, dass** die mechanische Aktion eine Perkussion der Außenwand des Schlauchs, der die hydraulische Verbindung gewährleistet und/oder die Anwendung einer Abfolge sinusoidaler Beanspruchungen variabler Frequenz auf die Außenwand des Schlauchs, der die hydraulische Verbindung gewährleistet, umfasst.

**3.** Verfahren nach einem der Ansprüche 1 bis 2, **dadurch gekennzeichnet, dass** die mechanische Aktion mit einer Diastole synchronisiert oder mit mehreren aufeinanderfolgenden Diastolen synchronisiert segmentiert wird.

**4.** Verfahren nach einem der Ansprüche 1 bis 3, **dadurch gekennzeichnet, dass**, wenn die Situation (b) ermittelt wird, das Verfahren die Korrektur des gemessenen Signals in Abhängigkeit von den dynamischen Parametern ($f_0$, z) der hydraulischen Verbindung (2) umfasst.

**5.** Verfahren nach einem der Ansprüche 1 bis 4, **dadurch gekennzeichnet, dass** es die Aufzeichnung des gemessenen Signals oder in der Situation (b) eines korrigierten Signals und/oder die Übertragung des Signals an ein Verarbeitungssystem umfasst.

**6.** Verfahren nach einem der Ansprüche 1 bis 5, **dadurch gekennzeichnet, dass**, wenn die Analyse des Frequenzinhalts des einfallenden Signals einen spektralen Schlupf und/oder eine Schwankung des relativen Gewichts der oberen Frequenzkomponenten ermittelt, der Schritt des Bestimmens der dynamischen Parameter ($f_0$, z) der hydraulischen Verbindung (2) derart durchgeführt wird, dass die Parameter erneut aktualisiert werden.

**7.** Verfahren nach einem der Ansprüche 1 bis 6, **dadurch gekennzeichnet, dass** es ferner die Ermittlung einer Entkoppelung zwischen dem zentralen arteriellen Druck und dem radialen arteriellen Druck des Patienten umfasst, wobei die Ermittlung die Durchführung einer tonometrischen Messung der humeralen/brachialen Arterie und/oder der Arteria carotis, die Überwachung der Geschwindigkeit der Ausbreitung der Pulswelle des Patienten, bestimmt ausgehend von der tonometrischen Messung und von dem gemessenes Signal, und den Vergleich der Geschwindigkeit mit einem Referenzwert umfasst.

**8.** Verfahren nach Anspruch 7, **dadurch gekennzeichnet, dass** bei Unmöglichkeit der Durchführung einer tonometrischen Messung der Arteria carotis der Aortendruck in Abhängigkeit von einer Transferfunktion ausgehend von der Messung des humeralen Drucks geschätzt wird.

**9.** Verfahren nach Anspruch 8 in Kombination mit Anspruch 10, **dadurch gekennzeichnet, dass** jeder der Schritte der Ermittlung einer Entkopplung durchgeführt wird und dass, wenn jede der beiden Ermittlungen eine Entkopplung des zentralen arteriellen Drucks vom radialen arteriellen Druck des Patienten ergibt, ein Alarm gesendet wird.

**10.** Vorrichtung (100, 200, 300) zur kontinuierlichen Überwachung der Messung des arteriellen Drucks durch einen Drucksensor (3), der über eine hydraulische Verbindung (2) mit einem zuvor in die Arterie eines Patienten eingeführten Katheter (1) verbunden ist, wobei die hydraulische Verbindung (2) an den Sensor (3) ein Signal des arteriellen Drucks des Patienten, bezeichnet als "einfallendes Signal", überträgt, das am intraarteriellen Ende des Katheters (1) anliegt, und der Sensor (3) das durch die hydraulische Verbindung (2) übertragene Signal in ein elektrisches Signal, bezeichnet als "gemessenes Signal", umwandelt, wobei die Vorrichtung umfasst:

    - ein Überwachungs- und Korrekturmodul (100), das mindestens einen Prozessor (101, 102) umfasst, wobei das Modul zur Durchführung der folgenden Schritte geeignet ist:

        - Bestimmen, ausgehend von dem gemessenen Signal, der dynamischen Parameter der hydraulischen Verbindung (2), wobei die Parameter die Eigenfrequenz ($f_0$) und den Dämpfungskoeffizienten (z) der hydraulischen Verbindung (2) umfassen,

- Analysieren des Frequenzinhalts des einfallenden Signals, um die zu übertragende maximale Frequenzkomponente (fh) des Signals zu bestimmen,
- Ermitteln, ausgehend von den dynamischen Parametern der hydraulischen Verbindung (2) und der zu übertragenden maximalen Frequenz, einer Situation aus den folgenden Situationen:

    (a) die Fähigkeit der hydraulischen Verbindung (2) zur Übertragung des einfallenden Signals an den Sensor (3) mit einer Verzerrung unter einem Grenzwert,

    (b) die Fähigkeit der hydraulischen Verbindung (2) zur Übertragung des einfallenden Signals an den Sensor (3) mit einer Verzerrung über dem Grenzwert und der Möglichkeit des Moduls, das gemessene Signal zu korrigieren,

    (c) die Unfähigkeit der hydraulischen Verbindung (2) zur Übertragung des einfallenden Signals an den Sensor (3) mit einer Verzerrung unter dem Grenzwert und der Unmöglichkeit des Moduls, das gemessene Signal zu korrigieren,

- eine an den Prozessor gekoppelte Mensch-Maschine-Schnittstelle, um, wenn die Situation (c) ermittelt wird, ein Warnsignal zu senden,
- ein Aktuatormodul (200), das zur periodischen Anwendung einer mechanischen Aktion auf die Außenwand des Schlauchs geeignet ist, der die hydraulische Verbindung (2) gewährleistet, wobei das Überwachungs- und Korrekturmodul dazu ausgelegt ist, das gemessene Signal als Antwort auf die mechanische Aktion zu analysieren und die dynamischen Parameter ($f_0$, z) der hydraulischen Verbindung ausgehend von der Analyse zu bestimmen,

wobei das Überwachungs- und Korrekturmodul dazu ausgelegt ist, die Ermittlung einer der Situationen (a), (b) oder (c) durchzuführen:

    (i) einen Vergleich des gemessenen Signals und eines korrigierten gemessenen Signals in Abhängigkeit von den dynamischen Parametern ($f_0$, z) der hydraulischen Verbindung (2),

    (ii) die Positionierung, auf einem Eigenfrequenz-Dämpfungs-Nomogramm, des repräsentativen Punkts der dynamischen Parameter ($f_0$, z) der hydraulischen Verbindung im Verhältnis zu einem von der maximalen Frequenzkomponente des verwendeten Signals (fh) bestimmten Toleranzbereich, und

    (iii) eine Überprüfung der Kohärenz der Ergebnisse der Schritte (i) und (ii).

11. Vorrichtung nach Anspruch 10, **dadurch gekennzeichnet, dass** das Überwachungs- und Korrekturmodul (100) umfasst:

    - einen ersten Prozessor (101), der zur Bestimmung der dynamischen Parameter ($f_0$, z) der hydraulischen Verbindung (2), Analyse des Frequenzinhalts des einfallenden Signals, Ermittlung einer der Situationen (a), (b) oder (c) und in der Situation (b) zur Festlegung der Korrekturparameter des gemessenen Signals in Abhängigkeit von den dynamischen Parametern ($f_0$, z) der hydraulischen Verbindung (2) geeignet ist,

    - einen zweiten Prozessor (102), der in der Situation (b) zur kontinuierlichen Korrektur des gemessenen Signals ausgehend von den vom ersten Prozessor (101) festgelegten Korrekturparametern geeignet ist.

12. Vorrichtung nach einem der Ansprüche 9 bis 10, **dadurch gekennzeichnet, dass** das Aktuatormodul (200) einen piezoelektrischen Betätiger oder eine elektromechanische Vorrichtung umfasst.

13. Vorrichtung nach einem der Ansprüche 10 bis 12, **dadurch gekennzeichnet, dass** sie eine drahtgebundene Verbindung zwischen dem Überwachungs- und Korrekturmodul (100) und dem Aktuatormodul (200) umfasst, wobei die drahtgebundene Verbindung geeignet ist, dem Aktuatormodul (200) ein Synchronisationssignal mit einer Diastole und/oder elektrische Energie vom Überwachungs- und Korrekturmodul (100) bereitzustellen.

14. Vorrichtung nach einem der Ansprüche 10 bis 12, **dadurch gekennzeichnet, dass** sie eine drahtlose Verbindung zwischen dem Überwachungs- und Korrekturmodul (100) und dem Aktuatormodul (200) umfasst, wobei die drahtlose Verbindung geeignet ist, dem Aktuatormodul (200) ein vom Überwachungs- und Korrekturmodul (100) gesendetes Synchronisationssignal mit einer Diastole bereitzustellen.

15. Vorrichtung nach einem der Ansprüche 10 bis 14, **dadurch gekennzeichnet, dass** das Aktuatormodul (200) zur festen Verbindung mit der hydraulischen Verbindung (2) beabstandet vom Überwachungs- und Korrekturmodul (100) geeignet ist.

16. Vorrichtung nach einem der Ansprüche 10 bis 15, **dadurch gekennzeichnet, dass** sie ein Verarbeitungssystem (300) umfasst, das zur Kommunikation mit dem Überwachungs- und Korrekturmodul (100) geeignet ist, wobei das Verarbeitungssystem (300) zur Verarbeitung des gemessenen Signals oder in der Situation (b) eines vom Überwachungs- und Korrekturmodul (100) übertragenen korrigierten Signals geeignet ist.

17. Vorrichtung nach einem der Ansprüche 10 bis 16, **dadurch gekennzeichnet, dass** sie ferner ein Gehäuse zum tonometrischen Messen einer humeralen Arterie und/oder einer Arteria carotis umfasst.

## Claims

1. A method of continuously monitoring the measurement of arterial pressure by a pressure sensor (3) connected via a hydraulic link (2) to a catheter (1) previously introduced into the artery of a patient, said hydraulic link (2) transmitting a patient's arterial pressure signal to the sensor (3), said "incident signal" applied to the intra-arterial end of the catheter (1) and the sensor (3) transforming the signal transmitted by the hydraulic link (2) into an electrical signal, said "measured signal", said method comprising the following steps, carried out by at least one processor (101, 102) of :

   - determination, from the measured signal, of the dynamic parameters of said hydraulic link (2), said parameters comprising the natural frequency ($f_0$) and the damping coefficient (z) of the hydraulic link (2),
   - analysis of the frequency content of the incident signal, so as to determine the maximum frequency component (fh) of said signal,
   - detection, on the basis of the said dynamic parameters of the hydraulic link (2) and the maximum frequency to be transmitted, of one of the following situations:

      (a) the ability of the hydraulic link (2) to transmit the incident signal to the sensor (3) with distortion below a threshold,
      (b) the ability of the hydraulic link (2) to transmit the incident signal to the sensor (3) with a distortion greater than said threshold and the possibility of correcting the measured signal,
      (c) the inability of the hydraulic link (2) to transmit the incident signal to the sensor (3) with a distortion below said threshold and the impossibility of correcting the measured signal,

   - if situation (c) is detected, an alert signal is sent via a man-machine interface,
   said method further comprising the periodic application, by an actuator module (200), of a mechanical action on the external wall of the pipe which provides the hydraulic link (2) and the analysis, by the processor, of the signal measured in response to said mechanical action in order to determine the dynamic parameters ($f_0$, z) of said hydraulic link,
   the processor implementing, for the detection of one of the situations (a), (b) or (c) :

      (i) a comparison of the measured signal and a measured signal corrected as a function of the dynamic parameters ($f_0$, z) of the hydraulic link (2),
      (ii) the positioning, on a natural frequency-damping graph, of the point representing the dynamic parameters (f0, z) of the hydraulic link in relation to a tolerance area determined by the maximum frequency component of the signal used (fh), and
      (iii) verification of the consistency of the results of steps (i) and (ii).

2. A method as claimed in claim 1, **characterised in that** said mechanical action comprises percussion of the outer wall of the tube which provides the hydraulic link and/or the application of a train of sinusoidal stresses of variable frequency to the outer wall of the tube which provides the hydraulic link.

3. Method according to one of claims 1 to 2, **characterised in that** said mechanical action is synchronised with a diastole or segmented in a synchronised manner with several successive diastoles.

4. Method according to one of claims 1 to 3, **characterised in that** in the event that situation (b) is detected, said method comprises correcting the measured signal as a function of the dynamic parameters ($f_0$, z) of the hydraulic link (2).

5. Method according to one of claims 1 to 4, **characterised in that** it comprises recording the measured signal or, in situation (b), a corrected signal and/or transmitting said signal to a processing system.

6. Method according to one of claims 1 to 5, **characterised in that** if said analysis of the frequency content of the incident signal detects a spectral shift and/or a variation in the relative weight of the higher frequency components, said step of determining the dynamic parameters ($f_0$, z) of the hydraulic link (2) is implemented so as to update said parameters.

7. Method according to one of claims 1 to 6, **characterised in that** it further comprises the detection of a decoupling between the central arterial pressure and the radial arterial pressure of the patient, said detection comprising the implementation of a tonometric measurement of the humeral/brachial and/or carotid artery, the monitoring of the propagation velocity of the pulse wave of the patient determined from said tonometric measurement and the measured signal, and the comparison of said velocity with a reference value.

8. Method as claimed in claim 7, **characterised in that** if it is impossible to carry out a carotid tonometric measurement, the aortic pressure is evaluated by a transfer function from the measurement of the humeral pressure.

9. Method according to claim 8 in combination with claim 10, **characterised in that** each of the said steps of detecting decoupling is carried out and **in that**, if each of the two detections reveals decoupling of the patient's central arterial pressure and radial arterial pressure, an alarm is emitted.

10. Device (100, 200, 300) for continuously monitoring the measurement of arterial pressure by a pressure sensor (3) connected, via a hydraulic link (2), to a catheter (1) previously introduced into the artery of a patient, said hydraulic link (2) transmitting to the sensor (3) a blood pressure signal from the patient, referred to as the "incident signal" applied to the intra-arterial end of the catheter (1), and the sensor (3) transforming the signal transmitted by the hydraulic link (2) into an electrical signal, referred to as the "measured signal", said device comprising :

    - a monitoring and correction module (100) comprising at least one processor (101, 102), said module being adapted to implement the steps consisting of:

        - determine, from the measured signal, the dynamic parameters of said hydraulic link (2), said parameters comprising the natural frequency ($f_0$) and the damping coefficient (z) of the hydraulic link (2),
        - analyse the frequency content of the incident signal to determine the maximum frequency component (fh) to be transmitted,
        - detect, on the basis of the said dynamic parameters of the hydraulic link (2) and the maximum frequency to be transmitted, one of the following situations:

            (a) the ability of the hydraulic link (2) to transmit the incident signal to the sensor (3) with distortion below a threshold,
            (b) the ability of the hydraulic link (2) to transmit the incident signal to the sensor (3) with a distortion greater than said threshold and the ability of said module to correct the measured signal,

            (c) the inability of the hydraulic link (2) to transmit the incident signal to the sensor (3) with a distortion below said threshold and the inability of said module to correct the measured signal,

    - a man-machine interface coupled to the processor to emit an alert signal if situation (c) is detected,
    - an actuator module (200), adapted to periodically apply a mechanical action to the external wall of the tubing that provides the hydraulic link (2), the monitoring and correction module being configured to analyse the signal measured in response to said mechanical action and to determine the dynamic parameters ($f_0$, z) of said hydraulic link from said analysis,

    the monitoring and correction module being configured to implement, for the detection of one of the situations (a), (b) or (c) :

        (i) a comparison of the measured signal and a measured signal corrected as a function of the dynamic parameters ($f_0$, z) of the hydraulic link (2),
        (ii) the positioning, on a natural frequency-damping graph, of the point representing the dynamic parameters (f0, z) of the hydraulic link in relation to a tolerance area determined by the maximum frequency component of the signal used (fh), and
        (iii) verification of the consistency of the results of steps (i) and (ii).

11. Device according to claim 10, **characterized in that** the monitoring and correction module (100) comprises :

    - a first processor (101), adapted to determine the dynamic parameters ($f_0$, z) of the hydraulic link (2), analyse the frequency content of the incident signal, detect one of the situations (a), (b) or (c) and, in situation (b), define parameters for correcting the measured signal as a function of the dynamic parameters ($f_0$, z) of the hydraulic link (2),
    - a second processor (102) adapted, in situation (b), to continuously correct the measured signal on the basis of the correction parameters defined by the first processor (101).

12. Device according to one of claims 9 to 10, **characterised in that** the actuator module (200) comprises a piezoelectric actuator or an electromechanical device.

13. Device according to one of claims 10 to 12, **charac-**

**terised in that** it comprises a wired link between the monitoring and correction module (100) and the actuator module (200), said wired link being adapted to supply the actuator module (200) with a signal for synchronisation with diastole and/or electrical energy from the monitoring and correction module (100).

14. Device according to one of claims 10 to 12, **characterised in that** it comprises a wireless link between the monitoring and correction module (100) and the actuator module (200), said wireless link being adapted to supply the actuator module (200) with a signal for synchronisation with a diastole emitted by the monitoring and correction module (100).

15. Device according to one of claims 10 to 14, **characterised in that** the actuator module (200) is adapted to be made integral with the hydraulic link (2) at a distance from the monitoring and correction module (100).

16. Device according to one of claims 10 to 15, **characterised in that** it comprises a processing system (300) adapted to communicate with the monitoring and correction module (100), said processing system (300) being adapted to process the measured signal, or, in situation (b), a corrected signal, transmitted by the monitoring and correction module (100).

17. Device according to one of claims 10 to 16, **characterised in that** it also comprises a box for tonometric measurement of a humeral artery and/or a carotid artery.

FIG. 1

FIG. 2

FIG. 3

FIG. 4

# FIG. 5

FIG. 6

## FIG. 7

## FIG. 8

EP 2 793 692 B1

FIG. 9

FIG. 10

FIG. 11

FIG. 12A

FIG. 12B

FIG. 13A

FIG. 13B

EP 2 793 692 B1

**FIG. 14**

**FIG. 15**

## RÉFÉRENCES CITÉES DANS LA DESCRIPTION

### Documents brevets cités dans la description

- DE 392799010
- US 2006064021 A1
- US 2010094113 A1
- DE 3927990

### Littérature non-brevet citée dans la description

- **M. CANNESSON.** Arterial Pressure Variation and Goal-Directed Fluid Therapy. *J Cardiothorac Vase Anesth,* 2010, vol. 24 (3), 487-497
- **J. MAYER ; J. BOLDT ; R. POLAND ; A. PETERSON.** G. Manecke, Continuous Arterial Pressure Waveform-Based Cardiac Output Using the FloTrac/Vigileo: A Review and Meta-analysis. *J Cardiothorac Vasc Anesth,* 2009, vol. 23 (3), 401-406
- **R. THIELE ; ME. DURIEUX.** Arterial waveform analysis for the anesthesiologist: past, présent, and future concepts. *Anesth Analg,* 2011, vol. 113 (4), 766-776
- **M. GARDNER.** Direct Blood Pressure Measurement - Dynamic Response Requirements. *Anesthesiology,* 1981, vol. 54, 227-236
- **C. PROMONET ; D. ANGLADE ; A. MENAOUAR ; S. BAYAT ; M. DURAND ; A. EBERHARD.** F. Grimbert, Time-dependent Pressure Distortion in a Catheter-Transducer System. *Anesthesiology,* 2000, vol. 92 (1), 208-218
- **M. ALLAN ; W. GRAY ; J. ASBUη.** Measurement of Arterial Pressure Using Catheter-Transducer Systems. *BrJ Anaesth,* 1988, vol. 60, 413-418
- **B. KLEINMAN.** Understanding natural frequency and damping and how they relate to the measurement of blood pressure. *J Clin Monit,* 1989, vol. 5 (2), 137-147
- **E. BILLIET ; F. COLARDYN.** Pressure measurement evaluation and accuracy validation: the Gabarith test. *Int Care Med,* 1998, vol. 24 (12), 1323-1326
- **A.W.PAULSEN.** Implications for clinical monitoring of intra-arterial blood pressure based on the frequency content of worst-case pressure waveforms. *Medical Instrumentation & Technology,* Mai 1993, 217-234
- **R. BROWER ; W. SPAANS ; P. REWIERSMA ; G. MEESTER.** A fully automatic device for compensating for artifacts in conventional catheter-manometer pressure recordings. *Biomed Eng,* 1975, vol. 10, 305-310
- **Y. KINEFUCHI ; T. SUZUKI ; M. TAKIGUCHI ; Y. YAMASAKI ; ET M. YAMAMOTO.** Evaluation of dynamic response of catheter-manometer systems for pulmonary arterial pressure. *J Appl Physiol,* 1994, vol. 77 (4), 2023-2028
- **A. ERCOLE.** Atténuation in invasive blood pressure measurement system. *Br J Anaesth.,* 2006, vol. 96 (5), 560-562
- Surviving Sepsis Campaign: international guidelines for management of severe sepsis and septic shock. **DELLINGER RP ; LEVY MM ; CARLET JM ; BION J ; PARKER MM ; JAESCHKE R ; REINHART K ; ANGUS DC ; BRUN-BUISSON C ; BEALE R.** 2008. Crit Care Med. Janvier 2008, vol. 36, 296-327
- Erratum. *Crit Care Med,* Avril 2008, vol. 36 (4), 1394-6
- **F. HATIB et al.** Peripheral vascular decoupling in porcine endotoxic shock. *J. Appl. Physiol.,* vol. 111, 853-860
- **H. FOK et al.** Régulation of Vascular Tone and Pulse Wave Velocity in Human Muscular Conduit Arteries - Sélective Effects of Nitric Oxide Donors to Dilate Muscular Arteries Relative to Résistance Vessels. *Hypertension,* Novembre 2012, 1220-1225
- **M. KANAZAWA.** Relationship between Aortic-to-radial Arterial Pressure Gradient after Cardiopulmonary Bypass and Changes in Arterial Elasticity. *Anestesiology,* Juillet 2003, vol. 99 (1
- **M. SHIMADA et al.** Estimation of Aortic-to-radial Artery Distribution of Arterial Wall Elasticity. *Tokai J. Exp. Clin. Med.,* 2008, vol. 33 (1), 1-5